# EUROPÄISCHE PATENTSCHRIFT

(11) **EP 1 296 964 B1**
(45) Veröffentlichungstag und Bekanntmachung des Hinweises auf die Patenterteilung: **11.04.2007**
(21) Anmeldenummer: 01960350.5
(22) Anmeldetag: 21.06.2001
(51) Int. Cl.: C07D 251/60

(54) **VERFAHREN ZUR HERSTELLUNG VON HOCH REINEM MELAMIN NACH TROCKENER AUFBEREITUNG**
METHOD FOR PRODUCING HIGHLY PURE MELAMINE AFTER DRY PREPARATION
PROCEDE DE FABRICATION DE MELAMINE ULTRA-PURE APRES TRAITEMENT SEC

(30) Priorität: 21.06.2000 DE 10030453
(43) Veröffentlichungstag der Anmeldung: 02.04.2003
(73) Patentinhaber: Bairamijamal, Faramarz, 4030 Linz (AT)
(72) Erfinder: Bairamijamal, Faramarz, 4030 Linz (AT)
(74) Vertreter: Herrmann, Uwe
(86) Internationale Anmeldenummer: PCT/EP2001/007039
(87) Internationale Veröffentlichungsnummer: WO 2001/098281

(56) Entgegenhaltungen:
- WO-A-97/47609
- WO-A-98/54160
- WO-A-98/55465

## Beschreibung

### Stand der Technik bei Melaminverfahren mit nasser Aufbereitung

Melamin wird bekanntlich nach drei Grundverfahren, katalytisches Niederdruckverfahren nach BASF oder Chemie Linz oder nach dem katalytischen DSM Mitteldruckverfahren, wie auch meist nach einem nicht katalytischen Hochdruckverfahren nach Nissan oder Montedison durch die Pyrolyse von Harnstoff unter Ammoniakatmosphäre erzeugt. In "Ullmann's Encyclopedia of Industrial Chemistry, Vol A 16, 5^{th} ed (1990), Seiten 171-185" sind die oben erwähnten Verfahren beschrieben.

In diesen Verfahren wird entweder das gasförmige Melamin, begleitet mit einem Trägergas (NH₃ gemeinsam mit den Reaktionsgasen NH₃/CO₂), durch die nasse Quenchung aus der Gasphase in die feste Phase überführt (ND- und MD-Verfahren). Alternativ wird auch oft die Melaminschmelze gemeinsam mit den Reaktionsgasen NH₃/CO₂ oder eventuell nach einer CO₂-Abtrennung durch CO₂-Stripping der Schmelze aus dem Hochdruckteil in einen Quencher entspannt (sog. HD-Verfahren mit nasser Aufbereitung). Aus dem Quencher wird stets eine Melaminsuspension abgezogen. Um ein reines Produkt, insbesondere für anspruchsvolle Anwendungsgebiete wie für die Lackindustrie zu erhalten, müssen weitere Aufbereitungsstufen durchgeführt werden.

Die im HD-Syntheseteil entstandenen NH₃/CO₂ Offgase werden in der Regel nach einer Kondensation zur wasserhaltigen Carbamatschmelze, wie z. B. in Atsumi Okamoto et al. in Hydrocarbon Processing, 70, November 1970 beschrieben ist, in die benachbarte Harnstoffanlage zurückgeführt, um daraus wieder Harnstoff zu erzeugen.

Bei dem katalytischen Niederdruckverfahren nach Chemie Linz erhält man das Melamin durch die Filtration der Melaminsuspension aus dem Quencher mit nachträglicher Trocknung.

Unabhängig von ND-, MD-, oder auch HD-Verfahren mit nasser Aufbereitung muß zur Erhaltung einer hohen Produktreinheit ein beträchtlicher Teil der Mutterlauge nach der Ausschleusung thermisch behandelt werden, um den Anteil an nicht ausreagiertem Harnstoff und die Melaminnebenverbindungen in die Ausgangsstoffe NH₃ und CO₂ zu zersetzen , wie sie z. B. in den Italienischen Patentanmeldungen Nr. 01282369 und Nr. 01282370 beschrieben sind. Der Anteil an Melamin und Melaminderivaten aus der ausgeschleusten Mutterlauge muß entweder vor der Eindampfung durch Neutralisation und Fällen eines Teils der Nebenverbindungen oder nach der Eindampfung wieder entfernt und umkristallisiert werden. Hierbei entsteht neben verfahrenstechnisch hohen Herstellkosten noch eine Abwasserproblematik, die durch eine eigens zu errichtende Anlageneinheit für die Abwasserbehandlung unter weiteren Energieeinsatz und Investitionsaufwand gelöst werden muß.

Das katalytische BASF-Verfahren erfordert eine Heißgasfiltration für Zurückhaltung des Katalysatorabriebes und Nebenverbindungen (Melem und Melam). Hier scheidet das feste Produkt durch Desublimation in einer Wirbelschicht aus.

Bei dem katalytischen Mitteldruckverfahren nach DSM muß ebenfalls das ganze Produkt nach dem Quenching durch Umkristallisation gereinigt werden, wodurch wieder hohe Herstellkosten und Abwasserprobleme mit einem hohen Produktionsaufwand entstehen. Auch hier muß zur Erhaltung eines verkaufsfähigen Produktes einen Teil der organisch belasteten Mutterlauge aus dem System ausgeschleust und einer Abwasserbehandlungsanlage zugeführt werden.

Ein wesentlicher Nachteil der Umkristallisation im Anschluß zu einem Quencher ist der damit einhergehende Ausbeuteverlust an Melamin. Der Grund liegt darin, daß der Betriebszustand des Quenchers (Druck und Temperatur) so hoch gehalten werden muß, daß der nicht ausreagierte Harnstoff in die Ausgangskomponenten NH₃/CO₂ zersetzt wird. Doch diese Prozeßführung verursacht gleichzeitig den Anstieg der unerwünschten Nebenverbindungen (Melamin-Hydrolyseprodukte). Das Ausmaß des Ausbeuteverlustes und Aufwand der nachfolgenden Reinigungsstufen ist hierbei höher als eine einfache Umkristallisation des Melamins aus dem festen Zustand. Denn bei der Umkristallisation in den obenbeschriebenen Verfahren wird ein Teil des umzukristallisierenden Melamins durch die Hydrolyse zu unerwünschten Nebenverbindungen umgewandelt, was wiederum durch eine erhöhte Ausschleusung an Kreislaufmutterlauge auskompensiert werden muß, damit die Produktqualität die Anforderungen erfüllt.

In den Hochdruckverfahren mit nassem Aufbereitungsteil wird aus dem Hochdruckteil entweder Melamin aus der Gasphase oder aus der flüssigen Phase in einen Quencher entspannt. Durch die Einhaltung des Betriebszustandes im Quencher kann man auch hier den noch nicht ausreagierten Harnstoff zum großen Teil hydrolysieren. Hier erhält man in der Regel eine Melaminsuspension mit einem Feststoffanteil von 5 - 10 Gew%, die durch Zumischung der Kreislaufmutterlauge in die Lösung überführt wird. Durch Alkalisierung der Melaminlösung kann man für die Einhaltung der Produktqualität einen Teil der unerwünschten (die Deammination-Nebenverbindungen) Nebenprodukte zu Melamin umwandeln. Der Verlustanteil an Melaminausbeute im Nassteil dieser Anlagen beträgt je nach geforderter Produktreinheit 2 bis 6 Massenanteil an Hauptprodukt aus dem Syntheseteil. Damit muß eine Mutterlauge in der Größenordnung von 2 bis 6 to Mutterlauge/to Melamin als ein organisch stark belastetes Abwasser aufbereitet und entsorgt werden.

Zur optimalen Betriebsbedingung bei den Hochdruckverfahren mit nassem Aufbereitungsteil ist man zur Erhöhung der Melaminqualität und damit Reduzierung der Energiekosten und Herabsetzen des Melamin-Hydrolyseverlustes, sowie Minimierung der Abwasserfracht bestrebt, die Melaminschmelze vor dem Quenching weiter mit NH₃ zu behandeln. Wie allgemein in der Melamintechnologie bekannt ist, kann man durch die Verschiebung der chemischen Gleichgewichtslage einen Teil der im Reaktor entstandenen NH₃ Partialdruck abhängigen unerwünschten Deammination-Nebenverbindungen nach einer gewissen Einwirkungszeit (Aiging der Melaminschmelze) zu Melamin umwandeln (Kirk-Othmer Encyclopedia of Chemical Technology, 4. Edition Vol. 4 p. 748-750). Diese Verfahrensstufe erfordert jedoch eine beinahe dreifache Druckerhöhung der Melaminschmelze von dem üblichen Synthesedruck zwischen 60 bis in der Regel 100 bar zu 180 bar bis 300 bar. Der Hydrolyseverlust und die Abwasserproblematik können dadurch nur vermindert werden.

Eine prägnante Zusammenstellung der verschiedenen nassen Verfahren im Vergleich zu Anlagen mit trockener Aufarbereitung unter Angabe der spezifischen Kennzahlen für den Energieverbrauch jedes Verfahrens sind in Patentschriften DE 35 00 188 C2 und US Nr. 568469 beschrieben.

Die obenbeschriebenen Probleme und insbesondere die Herstellkosten und verfahrenstechnisch bedingte aufwendige Offgasverarbeitung in den Normal- und Mitteldruckanlagen, wie auch die Einbindung der Offgase der Melamin HD-Anlage in die Harnstoffanlage haben in den letzten Jahren zu intensiver Forschung geführt. Unter anderem geht in DE 35 00 188 C2 die besondere Wirtschaftlichkeit der Melaminherstellung nach der trockenen Aufarbeitung hervor, wodurch sich auch Perspektiven auf Erschließung neuer Anwendungsgebiete und zusätzliche Konsumabsatzmärkte eröffnen.

### Stand der Technik bei Melaminverfahren mit trockener Aufbereitung

Die bisher veröffentlichten Publikationen konzentrieren sich auf das Ersetzen des wäßrigen Quenchmediums durch andere Kühlmedien, um die erhaltene Melaminschmelze aus dem HD-Syntheseteil ohne die Qualität beeinträchtigende Faktoren, wie die Bildung der störenden Deamminationsnebenprodukte Melem, Melam aber auch von noch nicht restlos umgesetzten Zwischenverbindungen, wie der Ausgangsstoff Harnstoff, und die Zwischenstufen Ureidomelamin, Cyanursäure, Ammelin und Ammelid zu verfestigen.

Als direktes Kühlmedium wird beispielsweise gasförmiges oder überkritisches NH₃ vorgeschlagen (WO 97/34879), was bei entsprechendem hohen Druck von 40 bis 1000 bar und hohen Temperaturen zu hohen Investitionskosten führt. Zur Abführung der Schmelze- und Kristallisationswärme ist hier eine große Gaskreislaufmenge erforderlich. Hierbei sind beispielsweise Sprühkristallisation (auch ohne Hilfsmittel, z. B. wie in US, 2,566,223 beschrieben) und Wirbelschichtverfestigung in Betracht gezogen (WO97/20826, WO98/52928).

Diese Verfahren sind jedoch durch verschiedene Nachteilen gekennzeichnet. Zum einen sind zur Aufrechterhaltung des Wirbelbett-, oder Fließbettzustandes, wie auch bei Sprühverfestigung mit Hilfsmittel, relativ hohe Gasmengen als Transport und Trägergas notwendig. Durch die relativ hohe Gasmenge (oder Kreislaufgasmengen) aber, worin Melaminstaub bei schwebendem Zustand für eine gewisse mittlere Verweilzeit aufgehalten werden muß, sind nun zum anderen relative große Apparate bei hohem Betriebsdruck und Betriebstemperaturen erforderlich. Unter Berücksichtigung der Betriebstemperatur führt die Auslegung zu sehr teueren dickwandigen Apparaten, was ein hohes Maß an Investition und Platz erfordert.

Ein anderer nachteiliger Faktor tritt bei Verfahren mit Kreislaufgas ein. Zum Kreislaufbetrieb des Trägergases ist bei entsprechendem Druck und Temperatur neben einem aufwendigen Spezialverdichter eine beträchtliche Verdichterleistung in mehreren Hundert Kilowatt Leistung zu erbringen. Das melaminhaltige Kreislaufgas muß vor der Verdichtung noch vom Melamin (respektive vom Partialdruck abhängigen Melamin und aerosoles Melamin) gereinigt werden, daß ein störungsfreier Betrieb möglich ist. Das betrifft auch in beschränktem Maße für melaminbelastetes Purgegas aus dem Kreislauf. Daher sind hier eine größere Anlageneinheit für Reinigung des Kreislaufgases und Kondensation der Purgegasmenge notwendig.

Auch die Verfahren unter Einsatz von flüssigem NH₃ als direktes Kühl- und Quenchmedium reduzieren zwar die anfallende Gasmenge bei der Verfestigung, erfordern jedoch trotzdem eine größere Anlageneinheit für die Behandlung des mit Melamin belasteten Kühlmediums. Betriebsstörungen infolge Produktagglomerisation, Anbackungen an Apparatewandungen und desgl. können nicht verhindert werden, so daß eine reduzierte Anlagenverfügbarkeit in Kauf genommen werden muß.

Als die aufwendigste Verfestigungsmethode sollte die direkte Kühlung der Schmelze unter Einsatz von festen kalten Melamin und festen kalten Inertstoffen in einer mit Kreislaufgas gebildeten Wirbelschicht (WO99/38852) erachtet werden.

Bei allen bisher weltweit in Betrieb befindlichen, wie auch bislang veröffentlichten Melamin Anlagen kann man stets das Hauptprodukt in einer einzigen Güteklasse erzeugen (starre Produktionslinie). Die Verfestigungsapparate bilden jeweils ein Hauptapparat, dessen redundante Ausführung aus Gründen der hohen Investition und der Vielzahl der Folgeapparate nicht realistisch ist. Ungeachtet der unterschiedlichen Anwendungsgebiete muß zwangsläufig die Produktionsstrategie auf die höchst mögliche Produktqualität gerichtet sein. Dies bedingt jedoch auch, aufwendige Produktionsstufen für ein mäßig anspruchsvolles Produkt durchführen zu müssen, denn die Produktionslinie erlaubt keine Differenzierung für eine mengen- und qualitätsorientierte Teilstromführung. Der wirtschaftliche Nachteil einer starren Melaminanlage liegt darin, daß der Kundenkreis einen mittleren und nicht angemessenen Preis entrichten muß, wobei der eine Kundenkreis auf Kosten des andern bedient wird.

### Gegenstand der Erfindung

Aufgabe der vorliegenden Erfindung ist es, ein Verfahren zur Melaminherstellung bereitzustellen, bei dem die Nachteile der Melaminherstellung nach nasser Aufbereitung vermieden werden und die trockene Aufbereitung so ausgestaltet wird, daß die aufwendigen und kostenintensiven Bedingungen für die Verfestigung der Melaminschmelze mit Kristallisationshilfsmitteln vermieden werden, und bei dem flexibel unterschiedliche Produktqualitäten bereitgestellt werden können.

Diese Aufgabe wird erfindungsgemäß durch ein Verfahren zur Herstellung von Melamin gelöst, bei dem durch nichtkatalytische Hochdruck-Pyrolyse (HD-Pyrolyse) von Harnstoff eine Melaminschmelze erhalten wird, die nach einer oder mehreren Raffinationsstufen und Kühlung unter simultaner NH₃-Partialsättigung durch direkte Hochdruck-Schmelzekristallisation (HD-Schmelzekristallisation) ohne Kristallisationshilfsmittel unter NH₃-Atmospäre bei indirekter Kühlung der Schmelze mit einem Kühlmittel oder einem Kältemittel verfestigt wird, das erhaltene Produkt unter Hochdruckbedingungen deodoriert und zur beliebigen Korngrößenverteilung in eine oder mehrere, vorzugsweise bis in vier Güteklassen finalisiert wird.

Bei dieser Erfindung handelt es sich um ein vorzugsweise kontinuierliches nicht katalytisches Hochdruck-Herstellverfahren zur Erzeugung von Melamin in vier verschiedenen Güteklassen, die in einer flexiblen Produktionslinie aus einer Anlage hergestellt werden können. Das hier angewandte Hochdruckverfahren basiert auf der sog. trockenen Aufbereitung, bei der das Melamin durch direkte Kristallisation aus der vorgekühlten Schmelze unter NH₃-Systemdruck auskristallisiert und nach Finalisierung das fertige Erzeugnis in beliebiger Korn- und Partikelgröße erhalten wird. Der Prozeß in der Kristallisationsstufe wird hier ohne Kristallisationshilfsmittel ausgeführt. Die Verfestigung wird oft mit flüssigem NH₃ als Quenchmedium z. B. in WO 98/27071, EP 0 808 836 A1, WO 97/47609, DE 35 46 893 C3, oder gasförmigem oder überkritischem NH₃ als Kühl-, Wirbel- und Transportmittel z. B. in WO 97/34 879, wie auch unter aufwendigen Bedingungen durch Einsatz von festen Kühlbeimitteln wie festem Melamin mit oder ohne Inertstoffen angewandt. Das vorliegende Verfahren mit der direkten Kristallisation ohne Kristallisationshilfsmittel zeichnet sich u. a. dadurch aus, daß der bislang niedrigste Investitionsaufwand unter dem Gesichtspunkt Produktherstellkosten bei mengen-, qualitäts- und bedarfsorientierter flexibler Produktionslinie erreicht wird.

Um eine hohe Produktausbeute und hohe Melaminreinheit zu erzielen, erweist es sich als besonders vorteilhaft, wenn die Melaminschmelze nach einem Primärreaktor und einem Abscheider in einer polytropischen CO₂-Stripperkolonne gestrippt und einem Sekundärreaktor bis zum vollständigen Umsatz von Harnstoff und Zwischenprodukten zugeführt wird. Die Melaminsynthese wird vorteilhaft bei 60 bis 400 bar und 350 bis 450°C und einer mittleren Verweilzeit zwischen 10 bis 500 min durchgeführt.

Da die konventionellen CO₂-Stripperkolonnen in der Melamin HD-Technologie bislang als isotherme Blasensäule-Apparate bekannt sind, erfordern sie zur Stripping eine relativ hohe NH₃-Strippgasmenge.

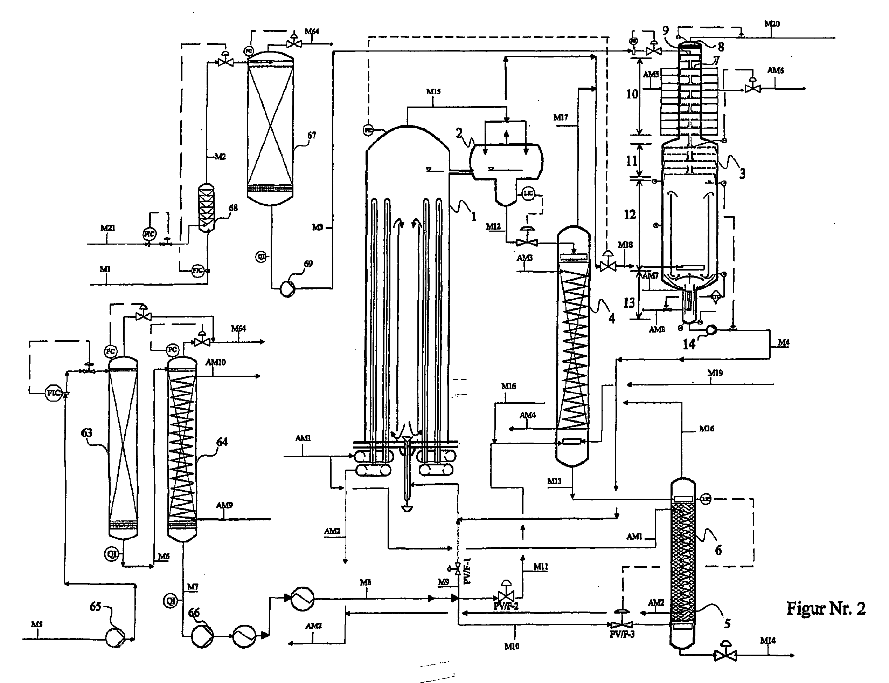

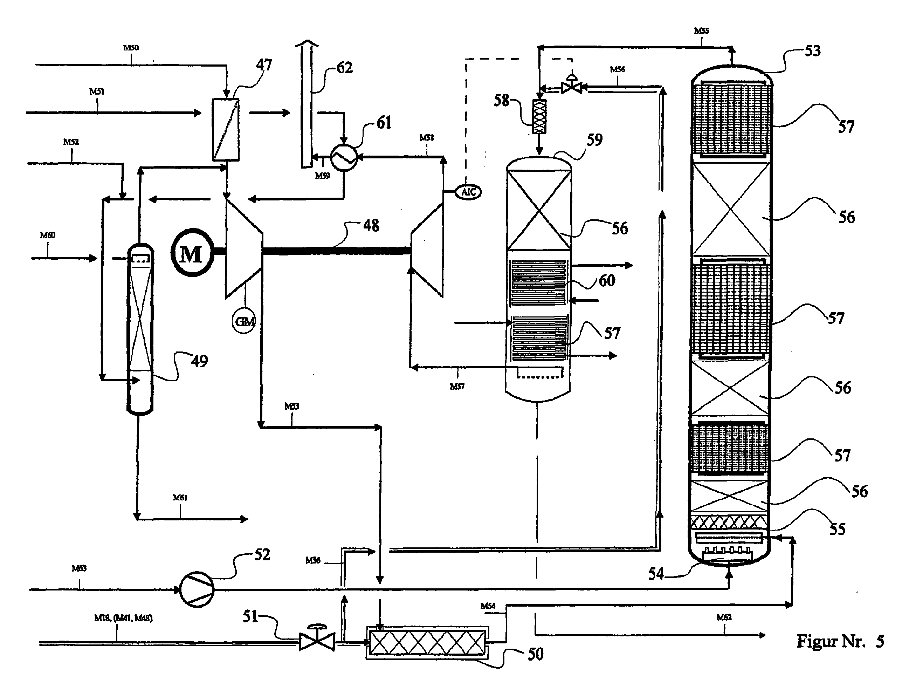

Es wird hier erwähnt, daß die hohe NH₃-Strippgasmenge auch eine unnötige Belastung der im Anschluß stehenden Offgaskolonne und danach die Offgas-Kondensationsapparete und Vorrichtungen in der Harnstoffanlage bewirkt. Insofern eine selbständige Melaminanlage mithilfe der Offgasincineration (nach Fig. 5) errichtet werden soll, muß aus wirtschaftlichen Gründen der Verbrauch an frischem NH₃ der Anlage minimiert werden. Diese Aufgabe wird hier erfindungsgemäß mit polytropischer CO₂-Stripperkolonne 4 so realisiert, daß der CO₂-Stripper mit Heizrohren ausgestaltet wird. Die CO₂-Stripperkolonne mit indirekter Beheizung 4 kann als eine Bodenkolonne oder besondersvorteilhaft als eine Packungskolonne mit integrierten Heizrohren in Fig. 2 konstruiert werden.

Der polytropische CO₂-Stripper ermöglicht neben dem verbesserten Stripping der Schmelze auch den Fortschritt der Harnstoffpyrolyse bei niedrigerem NH₃-Strippgasbedarf dadurch, daß der Durchbruch an nicht umgesetzten Harnstoff aus dem Primärreaktor 1 im polytropischen CO₂-Stripper 4 weiterreagieren kann. Für die Einhaltung der minimalen NH₃-Strippgasmenge wegen erweist sich auch die Packungskolonne unter Rieselbetrieb als besonders geeignet.

Die CO₂-Stripperkolonne im vorliegendem Verfahren unterscheidet sich von denen nach Stand der Technik dadurch, daß:
- die Kolonne zum CO₂-Stripping aus der Melaminschmelze mit den indirekten Heizrohren bestückt ist. Dadurch kann das Stripping forciert werden.
- Die Melaminschmelze strömt entweder über ein geeignetes Kolonnenpakkungsmaterial, in dessen Matrix die Heizrohre einzügig untergebracht oder sektionsweise angeordnet sind.
- Die polytropische CO₂-Stripperkolonne kann hier auch als Bodenkolonnne (Siebböden, Ventilböden, Glockenböden, Schlitzböden, Schlitzventilböden, desgl.) mit den aufliegenden oder in den Böden integrierten Heizrohren konstruiert sein.

Die langsameren Weiterreaktionen der reaktiven Zwischenstufen über Biuret, Triuret, Cyanursäure und derer Ammonisierung zum Melamin kann nach dem polytropischen CO₂-Stripper 4 in dem Sekundärreaktor 6 unter Einwirkung des NH₃-Gas in Gegenstromführung ausgeführt werden. Zur Erfüllung dieser Anforderung wurde hier erfindungsgemäß der Sekundärreaktor 6 durch spezielle Einbauten und Wärmeaustauschrohre 5 derart konzipiert, daß sich ein plug flow der beiden entgegenströmenden Reaktionsmedien, NH₃-Gas und die gestrippte Melaminschmelze ausbilden kann.

Die CO₂ gestrippte Melaminschmelze fließt hierbei in beinahe demselben Reaktionsdruck und derselben Synthesetemperatur zum Verteiler des Sekundärreaktors zu. Der Reaktor ist bis zur unteren Kante des Verteilers geflutet und wird in Zweiphasenbetrieb nach den Funktionsprinzipien einer beheizten gepackten Blasensäule betrieben.

Die Beheizung, vorwiegend mit Salzschmelze bei ca. 360 bis 460°C Salzschmelze Zulauftemperatur, kann in Gegenstrom zur Melaminschmelze oder besonders bevorzugt in Gleichstrombetrieb ausgeführt werden. Die Melaminschmelze fließt hier um die Heizrohre. Die Heizrohre des Sekundärreaktors 6 sind in parallel gerichteten statischen Mischungselementen derart eingesteckt und an den Header-Rohren geschweißt, daß sowohl ein plug flow Betrieb des aufwärts strömenden NH₃ Frischgases, als auch ein plug flow des entgegengerichteten abwärtsfließenden Melamin-Reaktionsmediums ermöglicht ist. Die Beheizung des Sekundärreaktors 6 in Gegenstrom- oder Gleichstromprinzip kann entweder Sektionsweise oder in einzügiger Ausführung erfolgen.

Die spezielle Packung 5 ist derart strukturiert, daß die abwärtsgerichtete Pfropfenströmung der Melaminschmelze unter ständiger Redispersion der Gasphase erfolgt. Die CO₂ gestrippte Melaminschmelze fließt unter Ausbildung einem Konzentrationsgradienten in einzügiger Führung durch den Sekundärreaktor.

Die Heizrohre und Mischungselemente sind in eine Matrix aus grobporigem Pakkungsmaterial 5 gegossen. Die Poren der Matrix, mit einem mittleren Durchmesser von 4 bis 5 mm, ermöglichen eine innige Durchmischung des Frischgases bei Unterbindung der Blasenkoaleszenz des aufsteigenden Gases. Die Frischgasmenge kann gegebenenfalls auch soviel gewählt werden, daß das gesamte Frischgas zum CO₂-Stripper 4 weiter geführt werden kann. Besonders vorteilhaft ist es, wenn das Abgas aus dem Sekundärreaktor 6 dem Frischgas zum CO₂-Stripper 4 zugemischt wird.

Durch diese spezielle Vorrichtung in einer derart gepackten Blasensäule ist gewährleistet, daß die axiale und radiale Dispersion beider entgegenströmender Phasen auf ein Mindestmaß reduziert wird. Nach dem Passieren des NH₃-Gasverteilers verläßt die Melaminschmelze mit einer Reinheit von 98 bis 99% den Sekundärreaktor.

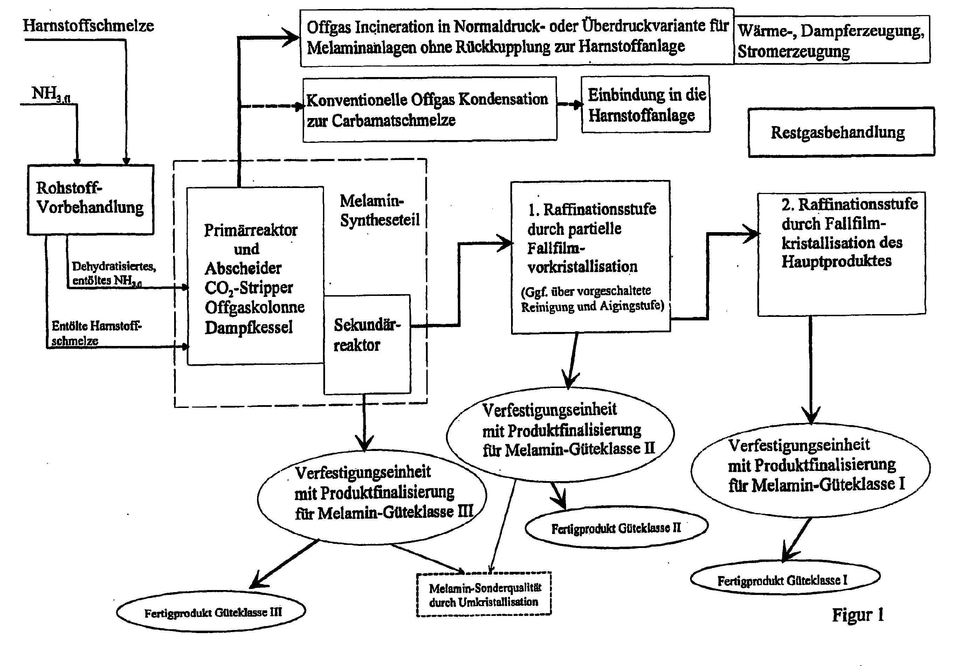

Bereits an dieser Stelle kann je nach Anforderung an die gewünschte Qualität und den Mengenbedarf ein beliebiger Anteil des Produktes zur Verfestigungseinheit eingeleitet werden, um ein Produkt in der Güteklasse III für zahlreiche Anwendungsgebiete bereitzustellen (entsprechend der Fig. 1 und 2). Falls ein Mengenanteil an Melamin mit höchster Reinheit in Sonderklasse verlangt wird, kann nach vorliegendem Verfahren diese Menge durch eine nasse Umkristallisation gemäß Fig. 1 aus dem festen Melamin bereitgestellt werden.

Bereits an dieser Stelle kann nach vorliegender Erfindung die Herstellung von Melamin derart flexibel ausgestaltet werden, daß das Produkt in der Güteklasse III durch die Verfestigung der Melaminschmelze nach dem Sekundärreaktor, oder eine Sonderqualität durch wäßrige Umkristallisation des unter Überdruck verfestigten Melamins hergestellt wird. In der Güteklasse III liegt die Reinheit vorzugsweise zwischen 98 und 99 Gew%, in der Sonderqualität vorzugsweise zwischen 99,0 und 99,5 Gew%. Da im Gegensatz zu den bisherigen Verfahren mit nasser Aufbereitung aus dem Quencher, die Umkristallisation aus dem festen Melamin bei einer Temperatur um ca. 100°C durchgeführt werden kann, fallen nun die nachteiligen Faktoren der nassen Aufbereitung (bei einem Quenchertemperatur von ca. 140°C oder gelegentlich höher) weit weniger ins Gewicht.

Nach Bedarf kann dem Sekundärreaktor eine Aigingstufe der Melaminschmelze mit oder ohne eine Reinigungsstufe nachgeschaltet werden, bevor die erste Raffinationsstufe der Melaminschmelze eingeleitet wird. Daher wird die Melaminschmelze vorzugsweise nach der HD-Pyrolyse in einer Reinigungsstufe gereinigt und durch eine Aigingstufe weiterbehandelt.

In dieser Erfindung wird zur Erfüllung der chemisch verfahrenstechnischen Aufgaben erstmals eine Offgaskolonne 3 in Fig. 2, unterteilt in 4 Zonen, 10, 11, 12, und 13, eingebracht.

Zur technischen Realisierung dieser Aufgabe wurde die Offgaskolonne so konzipiert, daß die heißen melaminhaltigen Reaktionsgase aus dem Primärreaktor und Strippergas aus der CO₂-Stripperkolonne in eine 4-Zonen-Offgaskolonne mit polytropischer Preabsorptionszone, adiabatischer Äerosolabscheidezone, autothermer Waschzone, und polytropischer Eindickerzone mit frischer Harnstoffschmelze abgekühlt und von Melamin gereinigt werden. Vorzugsweise findet die Offgasbehandlung bei 40 bis 400 bar und 138 bis max. 250°C statt.

Das Offgas passiert durch drei Zonen, während die aufgegebene entölte und stabilisierte Harnstoffschmelze am Kopf der Kolonne zuerst über nicht gekühlte Böden 9 zur Erreichung der Absorptionstemperatur der Harnstoffschmelze, anschließend über Böden 7 bestückt mit den Kühlrohren bis zum Sumpfteil 4 Zonen durchläuft und beladen mit suspendiertem Melamin, absorbierten CO₂ und NH₃ als Syntheseharnstoff über Reaktor-Feedpumpe 14 zum Primärreaktor 1 gefördert wird.

In der ersten Zone 10 findet die exotherme Preabsorption des gereinigten aus der 2. Zone 11 aufsteigenden Offgases in Harnstoffschmelze statt. Die exotherme Preabsorption des NH₃ und CO₂ unter teilweise Carbamatbildung in Harnstoffschmelze wird dabei unter simultaner Wärmeabführung auf jedem der Boden derart ausgeführt, daß die größte Wärmetönung durch den Absorptionsprozeß der Gase sofort am Entstehungsort über polytropisch gesteuerte indirekte Wärmeaustauschregister der Böden 7 abgeleitet wird.

Die abzuführende Wärme durch NH₃ und CO₂ Absorption, wie auch durch partielle Carbamatbildung kann zur Dampferzeugung (z. B. 5 bar Dampf) oder über einen Wärmeträger für andere interne Zwecke verwendet werden.

Damit wird bereits im Vorfeld zur Offgaswäsche und Offgaskühlung in der 2. und 3. Zone der Kolonne 11 und 12 der größte Teil der freiwerdenden Wärme mit dem gereinigten Offgas abgeführt. Die polytropische Prozeßsteuerung entlang der Preabsorptionszone kann entweder gradientenfrei oder bei unterteilten Kühlsektionen mit leichtem Temperaturgradienten, z. B. anhand der Temperaturmessung in Ablaufschacht des letzten Bodens der 2. Zone oder besonders günstig in Zulaufschacht des ersten Boden der 2. Zone durchgeführt werden. Bei unterteilten Kühlsektionen in der Preabsorptionszone kann z. B. die Temperatursteurng über den Ablaufschacht des entsprechenden letzten Bodens dieser Sektion vorgenommen werden. Das gereinigte Offgas verläßt die Kolonne über Tropfenabscheider 8.

Die streng definierte polytropische Prozeßführung in der Preabsorptionszone 10 wird nach erfindungsgemäßer Ausführung auch dadurch ausgezeichnet, daß hier eine genaue partielle Carbamatbildung in der 1. Zone um gerade soviel gefördert wird, daß in den folgenden Zonen 2 und besonders 3 endotherm die in situ Zersetzung erfolgen kann (siehe Prozeßbeschreibung der 3. Zone unter autothermer Prozeßführung).

In der zweiten Zone 11 der Kolonne fließt die mit CO₂ und NH₃ beladene Harnstoffschmelze unter teilweise gebildeten Carbamat über einige Böden. Durch diese Ausstattung kann im Betrieb das Ausmaß des mitgerissenen und ärosolen Melamins aus dem Blasensäuleteil (3. Zone) unterbunden werden.

In der dritten Zone 12 der Offgaskolonne wird das heiße mit Melamin beladenem Offgas von ca. 400°C und bis zu 6 Gew% gasförmigen Nebenverbindungen (hauptsächlich Melamin) bis auf ca. 200°C in einer autothermen Prozeßführung abgekühlt. Die hierbei abzuführenden Wärmen aus dem heißen Offgas und die Sublimationswärme des Melamins aus der Gasphase, wie auch die Kristallisationswärme des mitgerissenen Melamintröpfen werden nun in dieser Zone unter autothermen Bedingungen durch die Desorptionswärme des absorbierten NH₃ und CO₂, wie auch durch endotherme Zersetzungsreaktion des Carbamats zu gasförmigen NH₃ und CO₂ abgeführt.

Durch diese erfindungsgemäße autotherme Prozeßführung in der Offgaskolonne sind keine Wärmeaustauscher in der mit Melaminsuspension belasteten Harnstoffschmelze notwendig, da der Wärmeaustausch in situ durch Carbamatkonzentration und die Zersetzungsreaktion gesteuert wird. Die Offgaskolonne in der dritten Zone 12 wird durch die Hydraulik des Blasensäulebetriebes mit innerer Schlaufe charakterisiert. Durch den Schlaufenbetrieb wird das Medium, Harnstoffschmelze, Carbamat und Melaminsuspension in einer ständigen axialen Bewegung gehalten.

In der vierten Zone 13 wird ein Sedimentationskolonnenbetrieb derart realisiert, daß zum einen die Schlaufenströmung eine Klassierung des ausgeschiedenen festen Melamins im oberen Teil (entspricht dem unteren Teil der 3. Zone 12) bewirkt. Zum anderen wird eine indirekte Beheizung der Melamin angereicherten Harnstoffschmelze durchgeführt.

Der Ablauf des thermisch eingestellten Syntheseharnstoffes darf eine Temperatur von max. 250°C nicht überschreiten. Besonders vorteilhaft ist der Betrieb der Offgaskolonne bei demselben Synthesedruck im Bereich von 60 bis 150 bar zu betreiben. Die Betriebstemperatur liegt gewöhnlich bei 135 bis max. 250°C, besonders vorteilhaft zwischen 160 bis 220°C. Der Ablauf der Harnstoffschmelze aus der 1. und 2. Zone liegt ebenfalls in demselben Temperaturbereich, jedoch vorteilhaft bis max. 10°C unterhalb des thermisch eingestellten Syntheseharnstoffes in der Sedimentationszone 13.

Für die weitere Verarbeitung ist bei diesem Verfahren besonders vorteilhaft, daß die Melaminschmelze aus dem Sekundärreaktor durch eine zweistufige Raffination der Schmelze von den höher und niedriger als Melamin schmelzenden Verunreinigungen gereinigt wird. Die Raffinationsstufen werden vorteilhaft bei 20 bis 400 bar und 450°C bis zum Kristallisationspunkt der Schmelze bei entsprechendem NH₃-Partialdruck durchgeführt.

Bei der ersten Raffinationstufe 18 wird durch Fallfilmkristallisation eine partielle Kristallisation der Schmelze derart durchgeführt, daß großteils aller höher als Melamin schmelzenden unerwünschten Nebenverbindungen aus dem Hauptmassenstrom der Melaminschmelze entfernt werden. Die erfindungsgemäße Ausführung kann entweder mit einem Fallfilm-Vorkristallisator 18 für eine diskontinuierliche Prozeßführung oder mit zwei Apparaten für einen intermittierenden Betrieb gestaltet werden. Die Reinheit der Schmelze nach dem Fallfilm-Vorkristallisator liegt zwischen 98,5 bis 99,5 Gew%. Die beiden Raffinationsstufen 18 und 20 werden unter NH₃-Atmosphäre zum Schutz der Produktqualität vor der Nachbildung der Deamminationsnebenprodukten ausgeführt.

Die Raffinationsstufen finden besonders vorteilhaft bei gleichem Synthesedruck des Reaktors unter NH₃-Atmosphäre statt. Falls die Rafinationstufen bei vergleichbarem Synthesedruck praktiziert wird, kann das Restgas der Raffinationsstufe bevorzugt in den Offgaswäscher 3 abgeleitet werden.

Die Melaminschmelze erfährt bei dieser Verfahrensstufe entsprechend dem Systemdruck und damit dem absorbierten NH₃-Anteil eine minimale prozeßgesteuerte Abkühlung soweit, daß über dem NH₃ Partialdruck abhängigen Schmelzpunkt der Schmelze je nach ausgelegter Fallfilm-Rohrdimension ein Kristallwachstum der großteils höher als Melamin schmelzenden Verunreinigungen über eine Taktzeit von 20, besonders bevorzugt 12 Stunden an der Innenwand der Fallrohre ausgebildet wird.

Somit isoliert man die Verunreinigungen aus dem Vorkristallisator 18 in einer hohen Konzentration, bevor die Regenerationsphase eingeleitet wird. Diese Melaminschmelze mit niedrigerer Reinheit aus dem einen Kristallisator kann über ein Drukkerhöhungssystem wieder in den Primärreaktor zurückgeführt werden. Da bekanntlich die hoher als Melamin schmelzenden Komponenten hauptsächlich aus Melam und Melem bestehen, welche in Gleichgewichtsreaktion zu Melamin stehen. Durch die Rückführung dieser Komponenten kann über die chemische Gleichgewichtslage die Neubildung dieser Nebenverbindungen unterdrückt werden.

Zur intermittierenden Regeneration des Fallfilm-Vorkristallisators 18 werden erfindungsgemäß hier zwei Methoden bereitgestellt.

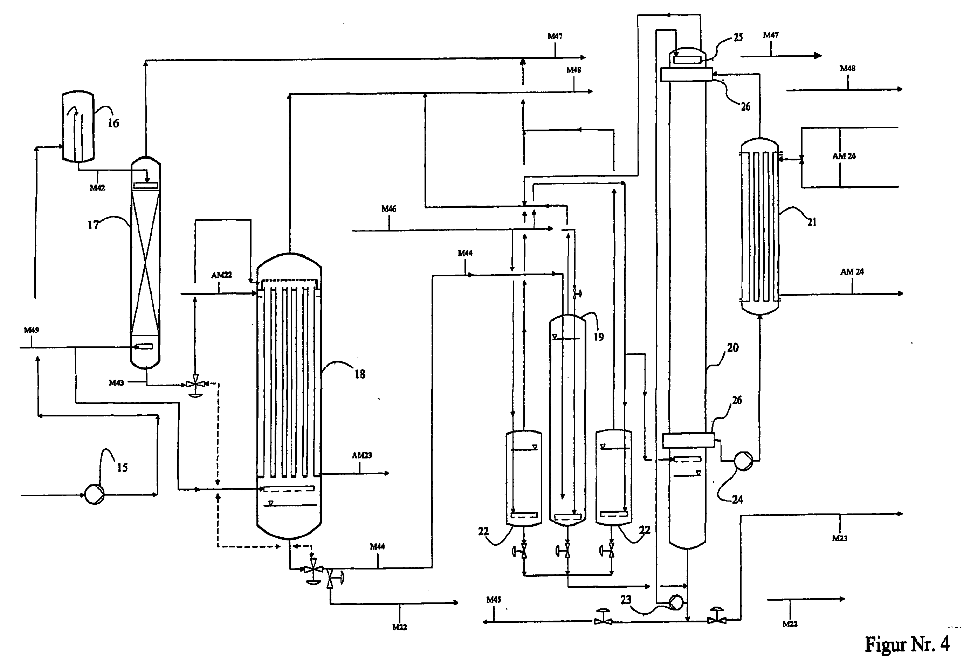

Die eine Methode bietet sich unter in sito Hydrolyse der Nebenverbindungen mit alkalischem wäßrigen Medium unter Hochdruckbedingungen des Kristallisators. Die andere Methode kann in der Weise in die Wege geleitet werden, daß der Vorkristallisator soweit aufgeheizt wird, daß die gesamte Nebenverbindungen abschmelzen. In der Vorlage trennt man sie aus dem Apparat, und unterzieht man in einem separaten Behälter (z. B. der Behälter 22 in der Fig. 4 bei der 2.Raffinationsstufe 20) ein Aiging der Verunreinigungen. Durch Recycling der Schmelze unter teilweise Ausschleusung kann die behandelte Melaminverunreinigung wieder zum Primärreaktor 1 zurückgeführt werden, um die Ausbeute der Anlage zu erhöhen.

Die über den Wärmeträger oder Salzschmelze abgeführte Wärme aus der Melaminschmelze wird jedoch hier nach erfindungsgemäßer Konzeption zur Vorwärmung, Verdampfung oder Überhitzung des frischen NH₃ eingesetzt. Das kann entweder für die NH₃ Syntheseammoniak oder für die Aufrechterhaltung der NH₃ Atmosphäre in den Apparaten eingesetzt werden. Falls der Verfahrensstufe, Fallfilm-Vorkristallisation 18 eine Aigingstufe 17 vorgeschaltet ist, kann bevorzugt das Restgas vom NH₃-Atmosphärengas aus dem Kristallisator als Feedgas für CO₂-Stripper 4 oder für den Sekundärreaktor 6 weiter verwendet werden.

Die zweite Stelle in dem Gesamtverfahren für die Erzeugung eines Teilproduktes oder des Gesamtproduktes in der Güteklasse II kann am Anschluß der Verfahrensstufe zur Fallfilm-Vorkristallisation nach Fig. 1 und 4 eingeleitet werden. Das Produkt an dieser Stelle weist eine erhöhte Reinheit in der Größenordnung von 98,5 - 99,5 Gew% auf und ist weitgehend von den Verunreinigungen wie Melem und Melam befreit.

Zur Realisierung der flexibelen Produktionslinien zur Erzeugung des Melamins in der Güteklasse II nach wird der Prozeß vorzugsweise derart ausgestaltet, daß das Produkt in der Güteklasse II durch die Verfestigung der Melaminschmelze nach der ersten Raffinationsstufe oder eine Sonderqualität durch die wäßrige Umkristallisation des unter Überdruck verfestigten Melamins hergestellt wird. Die Reinheiten liegen in der Güteklasse II vorzugsweise zwischen 98,5 und 99,5 Gew%, in der Sonderqualität vorzugsweise zwischen 99,0 und 99,5 Gew%.

Das Produkt mit der Güteklasse II kann nun für viele Anwendungen mit mittlerer bis höher Anforderung an Reinheit eingesetzt werden. Falls eine geringere Menge an Melamin mit höchster Reinheit verlangt wird, kann nach vorliegendem Verfahren diese Menge in Sonderqualität durch eine nasse Umkristallisation aus dem festen Melamin bereitgestellt werden. Die Erzeugung eines Produktes mit höchster Reinheit für Spezialanwendungen in der Güteklasse I kann nach vorleigender Erfindung dadurch erzielt werden, daß in der zweiten Raffinationsstufe 20 die Spuren an höher als Melamin schmelzenden und aller niedriger als Melamin schmelzenden Verunreinigungen aus der vorgereinigten Melaminschmelze entfernt werden.

Daher ermöglicht das vorliegende Verfahren, daß die Produktionslinie derart flexibel ausgestaltet werden kann, daß das Produkt in der Güteklasse 1 durch die Verfestigung der Melaminschmelze nach der zweiten Raffinationsstufe hergestellt wird. Die Reinheit liegt vorzugsweise zwischen 99,3 und 99,9 Gew%.

Die 2. Raffinationsstufe 20 wird zum Erhalten der Produktqualität vor der Nachbildung der Deamminationsnebenprodukte auch unter NH₃-Atmosphäre ausgeführt. Analog zu der 1.Raffinationsstufe 18 kann die Fallfilmkristallisation für einen Teil oder auch für den ganzen Produktstrom durchgeführt werden. Die 2.Raffinationsstufe (nach Fig. 4) wird unter ständiger Zirkulation mit einer Melaminschmelze-Kreislaufpumpe 23 bei einem Druck zwischen Synthesedruck und 20 bar, besonders bevorzugt bei 40 bis 90 bar und dem Kristallisationspunkt der verunreinigten Schmelze, jedoch zwischen 10°C über und 10°C unter dem Kristallisationspunkt des NH₃ Partialdruck abhängigen reinen Melamins ausgeführt.

Hier wird erfindungsgemäß die Umkristallisation des Hauptproduktes so durchgeführt, daß eine hoch reine Melaminfraktion an der Innenwand der Fallrohre auskristallisiert, während die Kreislaufmelaminschmelze zunehmend an Verunreinigungen angereichert wird. Die 2. Raffinationsstufe 20 wird daher bevorzugt so ausgeführt, daß eine vorliegende und vorgereinigte Melaminschmelze in ein Vorlagebehälter 19 unter NH₃-Schutzgas eingeleitet wird. Von dieser Vorlage wird in diskontinuierlicher Prozeßführung die Schmelze durch die Kreislaufpumpe 23 solange über den Verteilerboden 25 durch die Fallrohre beschickt, bis sich entsprechend dem Schmelzdiagramm eine gewisse Schichtdicke an Hauptprodukt an der Innenwand der Fallrohre ausgebildet hat.

Die Reinheit, wie bei allen Fallfilmkristallisatoren, wird durch die Temperatur-Zeit Steuerung bestimmt. Die Temperatursteuerung lehnt sich auch hier an den vom NH₃ Partialdruck abhängigen Kristallisationspunkt der Schmelze, so daß Ablauf des Taktzykluses der Kristallisator belegt ist und die reine Fraktion abgelassen werden kann.

Gemäß diesem Ausführungsbeispiel wird die Melamin-Schmelzezirkulation unterbrochen und die stark verunreinigte Fraktion in den einen der alternierenden Vorlagebehälter (Behälter 22 in der Fig. 4) abgeleitet. Durch teilweise Aufwärmung des Wärmeträgers mit dem Wärmeaustauscher 21 über die Wärmeträger-Kreislaufpumpe 24 und Verteiler 26 kann eventuell eine Erhöhung der Melaminreinheit dadurch erzielt werden, daß durch partielle Erschmelzung des Mischkristallisats eine Aufkonzentrierung der Verunreinigungen durchgeführt wird.

Nach diesem Takt wird nun durch Aufheizung des Wärmeträgers das gesamte hoch reine Melamin erschmolzen und in den Reinbehälter abgeleitet. Von dem Reinbehälter aus erfolgt wieder die kontinuierliche Melaminkristallisation über die Verfestigungseinheit.

Die Fallfilmkristallisation kann eventuell bei einer vorgeschalteten Aigingstufe 17 auch über dem Synthesedruck (mittels Pumpe 15) praktiziert werden, wobei in diesem Fall besonders vorteilhaft ist, daß das Restgas nach dem Kristallisator in den Syntheseteil zurückgeführt wird. Das Fertigprodukt nach der 2. Raffinationsstufe weist in der Güteklasse I eine Reinheit zwischen 99,3 bis 99,9 Gew% und erfüllt die Anforderungen für alle bekannten Spezialanwendungen.

Die kontinuierliche Hochdruck-Verfestigungseinheit des vorliegenden Verfahrens besteht vorzugsweise aus einem mehrstufigen Melaminschmelzekühler unter NH₃-Partialsättigung der Schmelze derart, daß die Melaminschmelze vor der HD-Schmelzekristallisation durch kontrollierte indirekte Kühlung unter kontrollierter simultaner NH₃-Partialsättigung bis zum NH₃-Partialdruck abhängigen Kristallisationspunkt (Stockungspunkt) der Schmelze vorgekühlt wird. Die NH₃-Partialsättigung liegt vorzugsweise zwischen 1 bis 99 % des NH₃-Sättigungsgrad der Schmelze.

Der Sättigungsgrad der Schmelze mit dem gasförmig zugemischten NH₃ erfolgt erfindungsgemäß in Abstimmung zu dem thermodynamischen Zustandspunkt der Schmelze mittelbar auf der Kristallisationsebene 34 des Produktkristallisators. Die simultane NH₃-Sättigung erfolgt sektionsweise 75 und wird genau mittels einer kontinuierlichen Messung, z. B. Viskosität oder Dichtemessung überwacht und gesteuert.

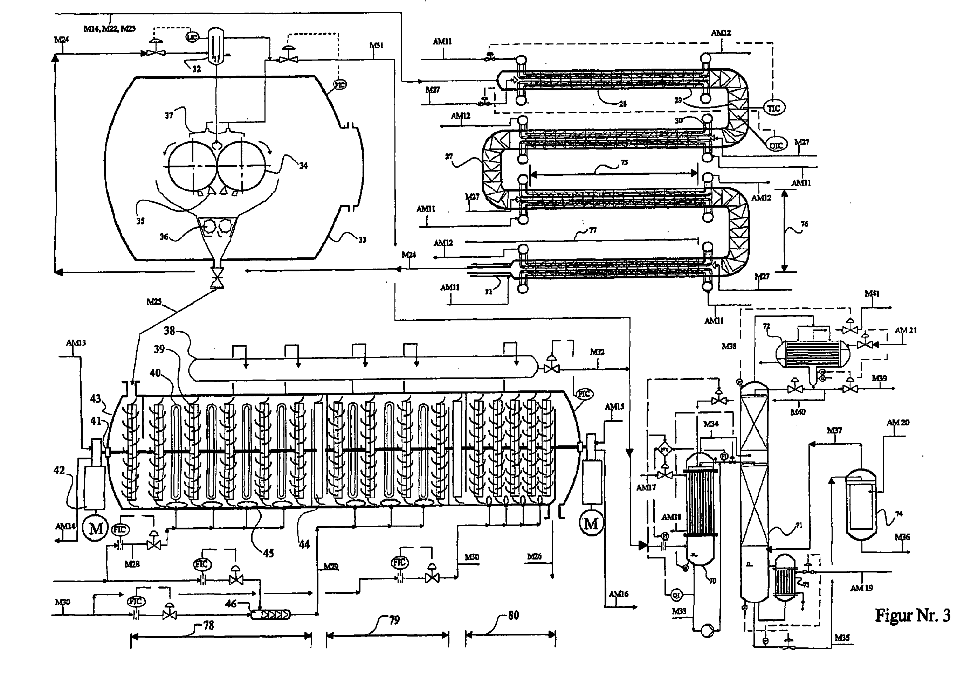

Die Systemgrenze dieses Schmelzekühlers 27 kann dank der besonderen Prozeßführung und Vorrichtungen des Schmelzekühlers (in Fig. 3) bis zum Stockungspunkt der Schmelze erstreckt werden. Um diese Aufgabe zu erfüllen, wurden erfindungsgemäß die Kühlrohren 28 mit statischen Mischungselementen 29 bestückt, und bei Sektionsweise geführten Prozeßsteuerung die NH₃ Gas über eine Perlgasdüse 30 bei erweiterter Mantelkühlung 31 entlang einer Beharrungsstrecke 77 bis zum Entspannungsgefäß 32 so konzipiert, daß die Schmelze bis auf den Kristallisationspunkt der Melaminschmelze vorgekühlt wird. Auf der Oberfläche des indirekt gekühlten Produktkristallisators 34 tritt nun mittelbar die spontane Kristallisation der Schmelze ein.

Die Verfestigung erfolgt nach diesem Verfahren vorzugsweise derart, daß die Melaminschmelze durch indirekte Kühlung mit einem Kühlmittel oder einem Kältemittel unter HD-Bedingungen und NH₃-Atmosphäre ohne Kristallisationshilfsmittel mithilfe eines Bandkristallisators, eines Einwalzenkristallisators oder besonders vorteilhaft mit einem Zwillingswalzenkristallisator zu einem porösen Melamingefüge verfestigt wird. Die Verfestigung der Schmelze wird vorteilhaft zwischen 1 bis 150 bar und einer Temperatur des verfestigten Melamins zwischen -120 bis 150°C durchgeführt.

In Ausführungsbeispiel wird der Prozeß so gesteuert, daß die Melaminschmelze nach der Entspannung in das Entspannungsgefäß 32 eine Temperatur bis in die Nähe des Stockungspunktes zur spontanen Kristallisation der Schmelze erreicht.

Der thermodynamische Zustandspunkt am Stockungspunkt der Melaminschmelze mittelbar auf der Oberfläche des Produktkristallisators 34 wird bei diesem Schmelzekühler besonders geeignet von der letzten Kühlsektion aus gesteuert (z. B. durch 4. Eintrittstelle des Wärmeträgers und die zugehörige NH₃-Perlgaseinleitung gemäß der Fig. 3). Auf der Kühlfläche des Produktkristallisators 34 (Bandkristallisator, Einwalzenkristallisator oder besonders geeignet der Zwillingskristallisator) erfolgt nun die Verfestigung unter Gasentweichung aus der Schmelze, so das sich eine poröse mit dem Schabmesser 35 leicht abkratzbare Melaminkristallisatschicht mit einer Dicke von ca. 5 mm ausbildet.

In Fig. 3 ist die Melaminschmelzekühler und der simultane partielle NH₃-Sättiger 27 exemplarisch mit 3 identischen und eine vierte in Prozeß ausgedehnter Kühlsektionen 75 mit exemplarisch 4 Prozeß in line gesteuerten Injektionsstellen für NH₃-Perlgas 30 dargestellt. Der Schmelzekühler in Fig. 3 ist auch exemplarisch mit 3 identische Relaxationsstrecken 76 gekennzeichnet, damit eine exakte Temperatur- und Qualitätsmessung zur präzisen Steuerung einer jeden Sektion gewährleistet ist.

Die vierte Relaxationsstrecke 31 ist dadurch gekennzeichnet, daß sie sich bis hin über das Entgasergefäß 32, in dem die letzte Qualitätsmessung zur Steuerung der Perlgasmenge durchgeführt wird, hinaus erstreckt und mittelbar auf der Kühlfläche 34 des Produktkristallisators endet. Auf der Schmelzeoberfläche -an der Aufgabestelle des Produktkristallisators- findet die letzte Temperaturmessung zur Steuerung der letzten Kühlsektion statt. Zuvor zwischen dem Entgasergefäß 32 und Verteilerlanze legt die Melaminschmelze die temperierte Beharrungsstrecke zurück. Die Steuerung der indirekten Beheizung und Temprierung der Melaminschmelze entlang der kurzen Beharrungsstrecke erfolgt ebenfalls über dieselbe -bevorzugt berührungslose- Temperaturmessung auf der Schmelzeoberfläche an der Aufgabestelle auf der Kühlfläche 34 des Produktkristallisators 33. Sie ist, wie oben erwähnt, die letzte Temperaturmessung des Schmelzekühlers und entspricht dem Stokkungspunkt der Melaminschmelze bei entsprechendem Verfestigungsdruck.

Die Kühlung des Melamins bis hin zum Stockungspunkt des reinen Melamins kann nach der 2. Raffinationsstufe 20 erstmals mithilfe dieses Schmelzekühlers 27 durchgeführt werden, da die Nebenverbindungen (insbesondere die höher als Melamin schmelzenden Deamminationsverbindungen) aus der Schmelze bereits entfernt worden sind, ohne daß es in einem kontinuierlichen Betrieb ein Blockieren des Apparates mit den höher als Melamin schmelzenden Nebenkomponenten bei längerer Betriebszeit eintritt.

Die NH₃-Perlgasmenge in jeder Sektion wird hier über eine Düse 30 in die Melaminschmelze feinperlig versprüht und durch die statischen Mischungselemente 29, die in den Kühlrohren 28 eingebettet sind, in die Schmelze homogen dispergiert. Die Zuleitung des NH₃ erfolgt stufenweise derart, daß keine starken Kavitationsimpulse auftreten. Die Anzahl der Sektionen liegen zwischen 2 bis 10, besonders geeignet werden in 2 oder 5 Sektionen ausgelegt. Die Kühlung der Melaminschmelze unter simultaner NH₃-Zumischung wird hier derart ausgeführt, daß die Absorptionswärme des eingebrachten NH₃ Gases in die Melaminschmelze durch die indirekten sektionsweise konzipierten Kühlrohre 28, die innerhalb des Schmelzekühlers 27 und statischen Mischungselementen 29 integriert sind, sofort abgeführt wird. Die Wärmeableitung kann nach dieser Erfindung in Gegenstrom-, jedoch besonders bevorzugt nach dem Gleichstromprinzip erfolgen. Hierbei fließt die Melaminschmelze um die Rohre 28 und wird unter ständiger Umleitung und Neuverteilung um die Kühlrohre gehalten.

Der NH₃-Sättigungsgrad in dem Melaminschmelzekühler richtet sich nach dem Arbeitsdruck der HD-Produktkristallisation im Hochdruckproduktkristallisator derart, daß die Schmelze nach dem Abfließen in den temperierten Entgasergefäß 32 und Lanzenverteiler während der Kristallisation mit leichter Ausgasung der zuvor absorbierten NH₃ begleitet ist. Dadurch erhält man eine poröse bis hoch poröse Kristallisatschicht, die leicht mit den Schabmessern 35 von der Kühlfläche 34 abgelöst werden kann.

Die Produktkristallisation unter Hochdruck im Hochdruckproduktkristallisator im Bereich von 1 bis 150 bar, insbesondere zwischen 20 bis 80 bar wird bei diesem Verfahren durch die rasche direkte Kristallisation aus der Schmelze bei indirekter Kühlung durch einen Wärmeträger als Kühlmittel oder besonders bevorzugt Mithilfe eines Kältemittels in Anlehnung an Fig. 3, bewerkstelligt. Als Produktkristallisatoren kommen in diesem Verfahren Bandkristallisator, Einwalzenkristallisator oder besonders bevorzugt ein Zwillingswalzenkristallisator 33 in Einsatz. Alle drei Kristallisationsapparate sind in Druckbehälter (Kristallisatorkammer) eingekapselt.

Das melaminhaltige Restgas aus der Schmelze wird über eine Lutte 37 gemeinsam mit dem Entspannungsgas aus dem Entspannungsgefäß 32 abgeleitet. Die Innenwand des Hochdruckproduktkristallisators 33 wird dabei so erwärmt, daß keine Taupunktunterschreitung des NH₃/Melamin Gasgemisch eintritt. Die beidseitig gelagerte Welle wird aus einer Seite entweder direkt (bei Einwalzenkristallisators) oder für den Zwillingwalzenkristallisator über eine Getriebe angetrieben. Auf der anderen Seite der Welle tritt Kühlmittel, bevorzugt Kältemittel über einen Düsensatz auf die Walzeninnenwand, während das verdampfte Kältemittel und Kondensat durch das koaxiale Austrittsrohr aus der Walze austreten. Die unterhalb der Aufgabestelle angeordneten Schabmesser 35 sorgen für gründliche Abtragung des Kristallisats, so daß saubere Kühlfläche 34 wieder für die Verfestigung zur Verfügung steht, ohne daß es sich Abnackungen und Agglomerate bilden.

Bei Einsatz eines Kältemittels, wie flüssiges NH₃ kann nach diesem Verfahren jede beliebige Walzeninnentemperatur bei entsprechendem Walzeninnendruck bis hin zu -33°C und atmosphärische Verdampfung eingestellt werden, womit ein Temperatursprung der Melaminschmelze bis zum verfestigten Melamin von ca. 408°C erzielt wird. Die Abkühlung erfolgt, wie es bei allen Walzen- und Bandkristallisatoren der Fall ist, derart rasch, daß sich keine Nebenverbindungen nachbilden können. Vorteilhaft ist jedoch, daß beispielsweise bei einem Kristallisatordruck von 60 bar in der Kammer eine Kammer-Innenwandtemperatur von 80°C nicht unterschritten wird, damit auf der Walze kein feuchtes Produkt und Agglomerisate über den weiteren Produktweg gebildet werden. Z. B. darf bei der minimalen Walzeninnentemperatur von ca. 75°C, respektive ein Walzeninnendruck von ca. 40 bar gewählt werden. Hierbei kann man die Kondensation des Kältemittels beispielsweise mit Kühlwasser über einen Sekundärkühlkreislauf recht einfach durchführen, ohne daß eine Kompression des Kältegases notwendig ist.

Bei Einsatz eines Wärmeträgers als Kristallisationskühlmittel kann jede beliebige Kühltemperatur bei entsprechender Fördermenge und Walzeninnendruck praktiziert werden. Das poröse verfestigte ammoniakhaltige Produkt wird mit den Schabmessern 35 von der Walze abgelöst und fällt dem thermostatisierten Nibbler 36 zu. Nach dem Nibbler besitzt das Grobgutprodukt je nach Ausführung eine mittlere Korngröße um 1 bis 3 mm. Das Grobgut kann nun in jeder Güteklasse entweder über Rohrweiche, Schneckenförderer oder besonders geeignet mittels Injektionsgas und Kreislauf einer pneumatischen Förderung zum Deodorationsapparat weitergeführt werden.

Das hoch reine Abwurfprodukt aus dem HD-Produktkristallisator ist porös und weist daher eine recht große Oberfläche auf. Das kalte und hoch poröse Produkt kann bei Einsatz eines Kältemittel eine Temperatur in der Großenordnung von 10 bis -30°C oder noch tiefer aufweisen. Daher enthält das Grobgut entsprechend dem angewandten Kristallisationsdruck eine gewisse Menge an adsorbiertem NH₃ und muß noch finalisiert werden.

Die Finalisierung des verfestigten Melamins erfolgt vorzugsweise derart, daß das verfestigte poröse Melamin zur Bereitstellung eines pH-neutralen und NH₃ geruchfreien Produktes durch Deodoration unter HD-Bedingungen und NH₃/N₂-Gemischgas in einem Dreikammer-Deodorations-apparat , oder dreizonigen Fließbettdeodorationsapparat oder zweizonigen Wirbelschicht-apparat behandelt wird, wobei die Deodoration bei einer höheren Temperatur als die Verfestigung des Melamins durchgeführt wird. Die Deodoration wird beispielsweise zwischen 1 bis 140 bar und einer Temperatur zwischen -120°C bis 1 °C unterhalb dem Schmelzpunkt des Melamins und einer mittleren Verweilzeit zwischen 1 bis 600 min in jeder Zone bei einem 90 bis 100 Vol% NH₃ in der ersten Aufheiz- und thermischen Desorptionszone, 10 bis 85 Vol% NH₃ und 90 bis 15 Vol% N₂ in der zweiten Mischgasstrippingzone und 0 bis 20 Vol% NH₃ und 80 bis 100 Vol% N₂ in der dritten Verdrängungsdesorptionszone durchgeführt.

Vor der Entspannung des Grobgutes auf drucklosen Zustand und Überführung zu den Mahlwerken muß der adsorbierte NH₃ unter strenger Einhaltung milder Betriebsbedingungen aus dem erstarrten Produkt entfernt werden. Die nachträgliche Deodoration des erzeugten Melamins ist auch insofern erforderlich, um ein pH-neutrales und vor allem ein geruchfreies Produkt für viele Anwendungsgebiete anbieten zu können.

Der Massenanteil des adsorbierten NH₃ ist um so höher, je höher der Kristallisationsdruck und je tiefer die Kristallisationstemperatur in der Kühlwalze gewählt wird und je höher die Porosität des Grobgutes ist. Da der Porositätsgrad des Grobgutes unmittelbar durch den Sättigungsgrad in den Kühl- und Sättigungsstrecken des Schmelzekühlers eingestellt wird, muß nun bei der Deodoration der Arbeitsdruck und die Desorptionstemperatur so gewählt werden, daß entsprechend der langsamen Diffusion des NH₃ eine produktschonende Deodoration ausgeführt wird.

Andererseits erfordert der durch Diffusion bedingte Entfernung des adsorbierten NH₃ relativ lange Verweilzeit, so daß die technischen Dimensionen eines atmosphärischen Deodorationsapparates derart groß werden, daß eine atmospärische Deodoration mit Strippluft bei mäßigen Betriebstemperaturen zu hohen Investitionskosten führt.

Um eine höhere Raum-Zeit-Ausbeute bei der Desorption des NH₃ aus Melamingrobgut zu erreichen, wird hier erfindungsgemäß die HD-Dreikammer-Deodorationsapparat 43 so konzipiert, daß die thermische Desorption und Verdrängungsdesorption des adsorbierten NH₃ aus dem Gefüge des Grobgutes eingeführt wird. Die HD-Deodorationsapparaten spielen insofern auch eine wichtige Rolle, daß die hohe Produktreinheit nach den Raffinationsstufen und Verfestigungseinheit während der thermischen Behandlung, durch z.B. Heiztaumeln mit Beschaufelung 39 und statische Heizringplatten 40 so erhalten bleibt, daß keine unerwünschten Deamminationsprodukte durch Unterschreitung des Gleichgewichtsdruckes zur Dissoziation des Ammoniaks aus dem Melamin eintritt *P_{NB₃}* (*Deodoration*) > *P_{NH₃}* (*Dissoziation*)*.*

Die obenerwähnten Randbedingungen haben hier erfindungsgemäß dazu geführt, daß die optimale Betriebsbedingungen unter Beachtung der Investitionskosten mit thermischer Desorption (entlang der Zone 1 der Fig. 3) und Verdrängungsdesorption (in der 2. Zone) Mithilfe Hochdruck-Dreikamme- oder dreizoniges HD-Fließbett oder zweizonigen HD-Wirbelschichtapparates entsprechend der geforderten Produktqualität und Mengenanteil gelöst werden konnte.

Die Desorption des adsorbierten Ammoniaks im kalten auskristallisierten Produktes ist hier verfahrenstechnisch bedingt und beschränkt sich dabei lediglich auf das Produktanteil, welches in den Güteklassen I, II und III nach direktem Kristallisieren erzeugt wurde. Der Anteil des Produktes, welches nach der Verfestigung einer wäßrigen Umkristallisation unterworfen wird, braucht nicht geruchfrei gemacht zu werden.

Die Anwendung der drei Verfahren richtet sich nach der gewünschten Güteklasse und kann unter Einhaltung des Mindestarbeitsdruckes *P_{NH₃}* (*Deodoration*) > *P_{NH₃}* (*Dissoziation*) in jedem beliebigen Temperaturbereich unterhalb der druckabhängigen Dissoziationstemperatur zur Entstehung der unerwünschten Deamminationsnebenverbindungen *T_{Deodoration}* < *T_{Dissoziation}* ausgeführt werden.

Es empfiehlt sich jedoch, die Desorptionstemperatur so hoch wie möglich zu wählen, um die Deodoration in einem möglichst kleineren und kostengünstigeren Apparat durchzuführen. Die Deodoration nach dieser Erfindung wird in Übereinstimmung mit den allgemeinen chemischen Regeln für Temperatursprung-Desorption und Verdrängungsdesorption ausgeführt, wobei die Diffusion des adsorbierten NH₃ aus dem Gefüge des porösen Melamingrobgutes der geschwindigkeitsbestimmender Schritt ist.

Hierbei muß stets der Druckbereich so gewählt werden, daß während der Deodoration keine Melaminqualität beeinträchtigenden Deamminationsnebenverbindungen entstehen. Grundsätzlich ist in der Melamintechnologie seit vielen Jahren die chemische Natur der Deamminationsnebenverbindungen bekannt, daß sie in gasförmigen, flüssigen und auch in festen Zustand durch Anwendung höherer Drücke und thermische Behandlung über eine gewisse Einwirkungszeit in das Hauptprodukt Melamin zurückgewandelt werden können (Kirk-Othmer Encyclopedia of Chemical Technology, 4. Edition Vol. 4 p. 748-750). Ebenso spielt die Hinreaktion zu den unerwünschten Deamminationsnebenverbindungen eine die Qualität beeinträchtigende Rolle, wenn die gewählte Temperatur-Druckpaarung nicht entsprechend der chemischen Gleichgewichtslage eingestellt wird.

Auf Hinreaktion des Melamins zu den unerwünschten Deamminationsverbindungen wird in zahlreichen Veröffentlichungen berichtet. Die Rückbildung der Deamminationsneben-verbindungen aus dem flüssigen Zustand zum Melamin wird z. B. in US Patent 2,566,227, aus dem festen Zustand bei ca. 250°C, Nippon Carbide Inc. Pat. 43-40895 und z. B. bei 300°C in Ciba, Pat. 523,448, wobei kristallines Melam (Kahlbaum) und melamverunreinigtes Melamin mit flüssigem NH₃ und festem melaminhaltigen Reaktionspartnern bei 250 bar durch die Gas-Feststoff-Reaktion zu Melamin umgewandelt wurde. Der Schmelzpunkt des niedriger als Melem schmelzenden Melamins bei demselben Druck liegt bei 306°C, wie in WO 97/20826 mitgeteilt wird.

Bei dem vorliegenden Verfahren kann das erfindungsgemäß durch das Deodorationsapparat (analog zu dreizonigem Fließbettdeodorationsapparat oder zweizonigem HD-Wirbelschicht-deodorationapparat) so ausgestaltet werden, daß die Prozeßführung und die apparativen Vorrichtungen bei kontinuierlicher HD-Deodoration nicht nur die Spuren der Nebenverbindungen zu Melamin zurückgewandelt werden, sondern auch gleichzeitig das Produkt so weit thermisch behandelt wird, daß in der darauf folgenden Schnittstelle des Apparates die Desorption des NH₃ unter Strippinggas (über den Statiskmischer 46, NH₃/N₂ und N₂) aus festem Melamin bewerkstelligt wird.

Hierfür passiert das Produkt durch die Vorrichtungen drei gasseitig voneinander getrennten Zonen durch die Trennbuffer mit beschaufelter Taumel 39 derart durch, daß das Grobgut eine genau definierte Aufheizungszone 78 (Zone 1) dann die Mischgas-Strippingzone 79 (Zone 2) und zum Schluß durch die gekühlte Reststrippingzone 80 (Zone 3) durchläuft.

In der Aufheizzone 78 wird das Produkt unter NH₃-Atmosphäre schonend durch die beheizte Taumel und statischen Heizringplatten 40 vorgewärmt, so daß die thermische Desorption unter milden Betriebsbedingungen ausgeführt werden kann. Das größte Anteil an adsorbierten NH₃ weicht hier aus dem Grobgut aus. Das NH₃ Schutzgas und das größte Teil des thermisch desorbierten NH₃-Gases weichen aus dem Grobgut über Dom 38 derart aus, daß sie durch mit Produkt gefüllten Buffer 44 nicht in die zweite Zone 79 gelangen können. Hierbei wird das Produkt innerhalb des Apparates mit dem Gas in Kontakt gehalten. Das NH₃-Schutzgas, NH₃/N₂-Verdrängungsgemischgas und N₂-Verdrängungsgas für dritte Zone 80 werden durch Halbmond-Filterkerzen 45 als Gasverteiler durch das Grobgut beschickt. Die Taumeln werden über die Welle (bestückt mit Labyrinthdichtung oder Gleitringdichtung 41) und außen angeordnetem Antrieb und Getriebesatz 42 angetrieben.

Hierbei ist wichtig, daß die Deodoration des mit adsorbierten NH₃ beladenen kalten Melamins so durchgeführt wird, daß keine Deamminationsnebenverbindungen während der Vorwärmung und der thermischen Desorption entstehen. Dafür kann z. B. die Vorwärmung bei einer Aufwärmrate zwischen 20 bis 100°C/min, vorteilhafter in 30 bis 50°C/min durchgeführt werden.

Damit die thermiche Desorption des adsorbierten NH₃ in ausreichendem Maße aus den Meso- und Mikroporen des Grobgutes -bedingt durch langsame Diffusion- erfolgen kann, wird in der 1. Deodorationszone 78 für lange Verweilzeit und homogene Durchmischung unter NH₃-Schutzgas gesorgt. Die mittlere Verweilzeit des Grobgutes in der thermischen Desorptionszone der Deodorationsstufe bei milden Betriebsbedingungen beträgt 1 bis 600 min, vorteilhaft zwischen 40 bis 300 min und besonders vorteilhaft zwischen 20 und 150 min. Die HD-Deodoration des MelaminGrobgutes kann in einem Druckbereich zwischen 1 bis 90 bar, vorteilhaft bei 20 bis 80 bar und besonders günstig bei 40 bis 60 bar durchgeführt werden. Die Temperatur-Druck-Verweilzeit Paarung erfolgt so, daß je rascher die thermische Desorption erfolgen muß, um so höher soll die Desorptionstemperatur eingestellt werden.

Dies nun erfordert einen höheren Systemdruck, damit die thermische Stabilität des festen Melamins gegen die Bildung von unerwünschten Deaminationsnebenverbindungen nicht beeinträchtigt wird. Aus demselben Grund wird in der Gasphase (unter Vernachläßigung des Melaminpartialdruckes) eine Schutzgasatmosphäre bis max. 100% NH₃, abzüglich des Melaminsdampfdruckes angestrebt.

Die Desorptionsstufe wird bei einer Systemtemperatur im Bereich 100°C bis Tₘₐₓ statt, wobei Tₘₐₓ maximal bis 1 °C unterhalb des Melamin Schmelzepunktes liegt. Je höher die Aufheizungstemperatur zur thermischen Deodoration gewählt wird, um so kurzer und einheitlicher soll die Verweilzeit gehalten werden, damit keine Beeinträchtigung der Produktqualität eintritt. Die entsprechenden Apparatedimenionen fallen unter diesen Bedingungen am minimalsten aus. Ein weiterer wesentlicher Vorteil der HD-Dreikammer-Deodoration 43 liegt darin, daß kein HD-Gebläse zur Deodoration erforderlich ist. Das geruchlose und pH-neutrale Produkt kann nach der Deodorationsstufe über intermittirende Zwischenbehälter entspannt und zum Melaminsilo abgeführt.

Das vorliegende Verfahren beinhaltet in seiner Geschlossenheit die Utility Einheiten für Offgasincineration (Fig. 5), Rohstoffvorbehandlung und Restgasaufbereitung (Fig. 3).

Zur Verarbeitung der Offgase der Melaminanlage in die Harnstoffanlage sind bereits verschiedene Verfahren und Veröffentlichungen bekannt. In der Regel werden dem Offgasstrom aus der Melaminanlage Kondensationshilfsmittel, meist wasserreiche Brüdenkondensaten aus dem Unter- und Normaldruckbereich der Harnstoffanlage oder notfalls Wasser (Atsumi Okamoto, Hydrocarbon processing No.70, Nov. 1970) zugesetzt.

Damit kann man die Offgase zu einer wasserhaltigen Carbamatschmelze kondensieren. Die hierbei abzuführende Carbamtbildungs- und Kondensationswärme wird oft zur Erzeugung von ND-Dampf verwendet. Der wesentliche Nachteil dieser Einbindungsvariante ist, daß durch das nötige Kondensationshilfsmittel ein zusätzlicher wäßriger Strom zuletzt in den Syntheseteil der Harnstoffanlage zugeführt wird, welches den Umsatzgrad des Harnstoffreaktors durch die Verschiebung der Gleichgewichtsreaktion herabsetzt.

Der weitere Nachteil ist, daß dieser zusätzlicher wäßriger Strom durch die energieintensiven Eindampfungsstufen der Harnstofflösung bis zum wasserfreien Harnstoff mitgeschleppt und verarbeitet werden muß. Der damit verbundene zusätzliche Energiebedarf stellt der Harnstoffanlage eine Belastung dar, der u. U. durch Kapazitätsverlust der Harnstoffanlage auskompensiert werden muß.

Die Rückführung der Melamin-Offgase in die Harnstoffanlage setzt gründsätzlich voraus, daß keine Kontamination des produzierten Harnstoffes mit dem krebsverdächtigten Melamin ausgeschlossen werden kann. Die Kontamination des produzierten Harnstoffes spielt insofern eine außerordentliche Rolle, da bekanntlich der Harnstoff als Dünge- und Füttermittel in großem Einsatz verwendet wird. Weiterhin hängt das Ausmaß der Wirtschaftlichkeit der Offgasenbindung in die Harnstoffanlage entscheidend von dem verfahrenstechnischen Stand der Harnstoffanlage ab.

Die Rückführung der Offgase setzt also prinzipiell neben einer benachbarten Harnstoffanlage auch den entsprechenden verfahrenstechnischen Stand der Harnstoffanlage zur Errichtung einer Melaminanlage voraus. Weiter ist der Betrieb der Melaminanlage vom Betrieb der Harnstoffanlage abhängig. Der Ausfall oder die Abstellung der Harnstoffanlage erfordert zwangsläufig die Abschaltung bzw. die Abstellung der Mealmainanlage. Ein Ausfall der Melaminanlage verursacht eine vorübergehende Betriebsstörung der Harnstoffanlage.

Der Gegenstand der neuen alternativen Offgasverarbeitung nach vorliegender Erfindung ist die vollständige katalytische Offgasverbrennung derart, daß die Offgase nach der Offaskolonne einer Incinerationstufe unter Normaldruck oder Überdruck zugeführt werden, wobei die gewonnene Wärme für interne Zwecke in der Anlage oder für externe Einsätze, vorzugsweise für die Dampferzeugung, den Antrieb der Arbeitsmaschinen oder für die Erzeugung elektrischer Energie, verwertet wird.

Sie kann insbesondere für die Standorte in Betracht kommen, wo die Harnstoffversorgung, u. U. auch eine preisgünstige Harnstofflieferung gegeben ist, jedoch keine Harnstoffanlage am Standort zur Verfügung steht. Durch die Anwendung der vorliegenden Verfahrensstufe zur Offgas Incineration kann nun den Sachleistungsunternehmern ohne Harnstoffanlage eine Melaminanlage angeboten werden.

Durch die vorliegenden Verfahrensstufe kann auch für Betreiber derjenigen Melaminanlagen ein Umbau der weniger wirtschaftlichen Offgaseinbindung in die Harnstoffanlage zur Offgas Incineration ermöglicht werden.

Da bei der vollständigen Offgasverbrennung mehr Energie frei wird, als sie allein für die energetische Versorgung der Melaminanlage an Wärme benötigt wird (zur Erhitzung der Salzschmelze für die Synthese, Erhitzung oder Verdampfung des Wärmeträgeröls, zur Dampfenergie und Stromgewinnung für elektrische Aggregate), kann mit Incineration zusätzliche Energie gewonnen werden. Diese Zusatzenergie kann z. B. für die Dampfversorgung des Werkes, zur Stromerzeugung oder zum Einsatz in den anderen Anlagen verwertet werden. Beide Verfahrensvarianten (Normaldruck- oder Überdruckincineration) nutzen nicht nur den Wärmeinhalt der Offgase durch Verbrennung, sondern auch die im Offgas enthaltene Druckenergie, welche über die Expanderturbine beim Prozeß verwertet wird.

Neben den obenerwähnten Möglichkeiten erlaubt die Offgasincineration auch das Luftstripping 49 der meist schwach amminiak- oder ammoncarbonathaltige Abwässer, oder Aufbereitung sonstiger mäßig wertvollen Medien. Hierfür kann die erwärmte Verbrennungsluft vor der Kompression mit dem Maschinensatz 48, gegebenenfalls bei weiterer Vorwärmung durch Bypaß-Strom um den LUVO 61, zuerst als Strippluft für diese Medien verwendet werden. Auch die NH₃-, melaminhaltige oder sonst belastete Abluft aus der Anlage kann hier mitverarbeitet werden, ohne die Incineration nur auf Offgase der HD-Melaminanlage einzuschränken.

Die zur Verbrennung erforderliche Prozeßluft nach dem Luftfilter 47 kann ggf. gemeinsam mit der Abluft aus der Anlage (ggf. nach einer Feststoffiltration) zur Saugseite eines Verdichters, z. B. ein Axialverdichter mit verstellbaren Leitschaufeln oder einer Gebläse zugeführt und auf den entsprechenden Verbrennungsdruck gebracht werden. Die heißen Offgase werden nach der Entspannung 51 auf Verbrennungsdruck einem Statikmischer 50 zugeleitet. Ein geringer Teil der Offgase wird für die selektive katalytische Reduktion der NOx-Gase nach dem Incinrationreaktor 53 zum SKR-Reaktor 59 abgezweigt.

Nach der homogenen Vermischung der Verbrennungsluft mit dem zu verbrennenden Offgas wird das Gasmedium in den Incinerationreaktor 53 eingeleitet, der in der Anfahrphase mit dem Anfahrerdgas durch 52 auf die Reaktionstemperatur gebracht wurde. Die Erdgasbrenner 54 können dabei nicht nur als Anfahrvorrichtung, sondern auch für Lastausgleich und Zusatzleistung der Verbrennung fungieren.

Nach einem Verteiler und Gleichrichter 55 passiert das Verbrennungsgemisch die erste Katalysatorschicht 56 und erfährt dabei die erste Überhitzung. Die Katalysatorschicht als Festbett kann hier in jeder beliebigen Ausführung gestaltet sein. Nach dem ersten Festbett wird ein Teil der Verbrennungswärme an den ersten Wärmeaufnehmer 57 so weit abgegeben, daß das Reaktionsmedium nicht in das Löschgebiet gelangt. Der Vorgang wiederholt sich bei abgestimmter Katalysator-Festbetthöhe 56 und entsprechender Wärmeaustauschfläche des Wärmeverbrauchers 57 beliebig oft, so daß nach der letzten Stufe das NOx haltige Restgas die entsprechende Eintrittstemperatur zum SKR-Reaktor (selektive katalytische Reduktion) 59 beibehalten kann.

In Abstimmung mit dem NOx-Gehalt der Entspannungsgase in den Kamin 62 wird die erforderliche Injektions-NH₃ für den SKR-Reaktor 59 über entsprechenden Statikmischer 58 zudosiert. Die Restwärme des Rauchgases gemeinsam mit der Wärme durch die selektive katalytische Nachoxidation wird schließlich dem letzten Wärmeverbraucher, z. B. einem Economiser 60 abgegeben.

An dieser Stelle unterscheidt sich die ND- von der ÜD-Incineration dadurch, daß bei ÜD-Incineration der obere Heizwert des Rauchgases ausgenützt wird, während bei ND-Incineration diese Wärme über den Expander 48 und Kamin 62 als zusätzliche Kaminverlust in die Atmosphäre abgegeben wird. Nach der Entspannung der Rauchgase über die Expanderturbine des Maschinensatz 48, wo auch z. T. die Druckenergie des Offgases ausgenützt wird, gelangt das gereinigte Rauchgas über den Kamin in die Atmosphäre.

Die Wärmeverbraucher 57 im Incinerationreaktor 53 und SKR-Reaktor 59 können zur Erhitzung der Salzschmelze, Wärmeträgeröl, NH₃-Erhitzer und Überhitzer für die Melaminanlge, aber auch zur Erzeugung von Dampfenergie als Sattdampf oder überhitzter Dampf für eine Dampfturbine zum Antrieb einer Arbeitsmaschine oder zum Generator für Erzeugung der elektrischen Energie eingesetzt werden.

Die Offgas-Incineration nach Fig. 5 dieses Verfahrens kann im Druckbereich zwischen 1 bis max 150 bar, besonders im Bereich von 1 bis 10 bar für ÜD-Incineration und im Bereich 1 bis max. 5 bar für ND-Incineration ausgeführt werden.

Um bei einem Melaminherstellverfahren mit trockener Aufbereitung ein Produkt in hoher Reinheit zu erhalten, sind neben den oben eingeführten Synthese- und Raffinationsschritten der Einsatz von hoch reinen Rohstoffen unerläßlich. Insbesondere soll die Auswirkung der mit Produkt kontaktierenden NH₃-Betriebsmittelgase (NH₃-Zusatzgas, NH₃-Reaktionsgas, -Strippgas, evtl. Aiginggas, -Schutzgas sowie NH₃-Perlgas und NH₃-Deodorationsgas) mit den eingebrachten Wasserspuren unter bunden werden, damit es keine unerwünschten Hydrolyse-Nebenverbindungen aus Melamin entstehen können.

Ein weiterer Grund für Einsatz reiner Rohstoffe ist durch die relativ hohe Pyrolysetemperatur bedingt, welche die Bildung von Foulingsprodukten (z. B. Ausbrand der verschleppten Ölspuren an den Heizrohren und desgl.) fördert. Die Vorreinigung der Rohstoffe verringert auch insofern die Investitionskosten, daß der zuzuschlagende Foulingsfaktor für Dimensionierung der wärmeaustauschenden Apparate (1, 3, 4, 6, 18, 20 und 27) geringer gewählt werden kann.

Daher ist zur Erzeugung eines reinen Produktes besonders vorteilhaft, daß die Einsatzstoffe für die Melaminherstellung, Harnstoff und Ammoniak, vor dem Melaminprozeß entölt werden, wobei der Ammoniak zusätzlich dehydratisiert wird.

Die H₂O-Komponente der Harnstoffschmelze wird bekanntlich in der Offgaskolonne durch die Harnstoffhydrolysereaktion zerlegt, so daß keine H₂O-Komponente über den Harnstoffweg in den Primärreaktor gelangen kann.

Gemäß Fig. 2 wird der Flüssigammoniak aus dem Versorgungsnetz oder aus dem Vorlagebehälter mit entsprechender Zwischenspannung zur Desorption der in NH_{3,fl} absorbierten Inertgase wie z. B. H₂, N₂, CH₄ , falls erforderlich über Boosterpumpe 65, zur Adsorberkolonne 63 für die Entfernung der Ölspuren und zum Dehydratationsadsorber 64 in beliebiger Reihenfolge der Adsorberkolonnen beschickt.

Bevorzugterweise wird die Adsorberkolonne zur Ölentfernung 63 so ausgelegt, daß der Adsorbens für mindestens eine Reisezeit der Anlage ausreicht, um die Kolonne während der Abstellungszeit vom neuen zu befüllen, da die Regeneration des Adsorbens in der Regel gegenüber der Entsorgung nicht rentabel ist. Als Adsorbentien kommen bei diesem Verfahren Aktivkohle, Aktivtonerde, Molekülarsieb oder Pillard Clays Adsorbens in Einsatz.

Der Dehydratationsadsorber 64 mit speziellem Molekularsieb, oder Silikagel wird bevorzugt zweifach ausgeführt, damit eine intermittierende Regeneration durch die thermische Desorption (mit indirektem Heizkorb im Adsorberbett) während des Normalbetriebes möglich ist.

Als Qualitätsmeßgröße bei der Adsorberkolonne zur Ölentfernung kommen Trübungs-messung oder sonstige difraktometrische Messungen und beim Dehydratationsadsorber können eine Leitfähigkeitsmessung oder ionensensitive Elektroden eingesetzt werden. Das für den Prozeß vorbereitete flüssige NH₃ aus der NH_{3,fl} Hochdruckpumpe 66 ist weitgehend inerten, öl- und wasserfrei.

Hierfür wird in der Harnstoffschmelzeleitung zuerst geringfügige Mengen an NH₃ Gas zur Stabilisierung der Schmelze gegen vorzeitige Bildung von Biuret und Triuret bei entsprechender Verweilzeit in der Adsorberkolonne 67, Hamstoffpumpe 69 und den Rohrleitungen eingeleitet. Hierzu wird durch ein Dispergierorgan (ggf. mit Schmelzekühlung zur Abführung der Absorptionswärme des NH₃ in Harnstoffschmelze) 68 gemäß der Fig. 2 das NH₃ Stabilisationsgas in die Harnstoffschmelze fein verteilt. Die Schmelze fließt in die Adsorberkolonne 67 unter Rieselbetrieb oder voller Beaufschlagung des Adsorberbettes und passiert nach feinem Edelmetallfilzmaterial zur Zurückhaltung aller Abriebstoffe zur Harnstoff-Hochdruckpumpe 69.

Als Adsorbentien werden hier Aktivkohle, Aktivtonerde oder Molekularsiebe angewandt. Da eine Regeneration des beladenen Adsorbens auch hier nicht wirtschaftlich ist, wird die Kolonne so ausgelegt, daß sie für mindestens eine Reisezeit der Anlage ausreichend dimensioniert ist.

Analog zu den Melaminherstellverfahren mit trockener Aufbereitung unter Einsatz von Kristallisationshilfsmittel wie flüssiges, gasförmiges oder überkritisches NH₃, muß auch nach dem vorliegenden Verfahren die melaminhaltigen Restgase aus dem System abgezogen und einer Aufbereitung unterzogen werden.

Angesichts der wesentlich niedrigeren Restgasmengen nach vorliegender Erfindung erweist es sich als besonders günstig, wenn die melaminhaltigen HD-Restgase aus der Anlage entweder der Incinerationstufe zugeführt werden oder durch Fraktionierung in eine reine NH₃-Fraktion und Pulvermelamin zerlegt werden. Das erhaltene Pulvermelamin kann der Deodorationsstufe zugeführt werden.

Die Behandlung des mit Melamin beladenen Restgases aus der Verfestigungseinheit und Purgegas der Deodorationsstufe, sowie Rest- und Schützgase aus den Raffinationsstufen (sofern sie entsprechend dem gewählten Systemdruck nicht in Offgaskolonne eingeleitet werden), können in der Anlageneinheit für Restgasbehandlung aufbereitet und die erhaltenen fraktionierten Wertstoffen wieder in den Prozeß zurückgeführt werden.

Der markante Unterschied bei diesem erfindungsgemäßen Verfahren gegenüber den in der Einleitung erwähnten Restgasaufbereitungsverfahren liegt darin, daß hier durch die direkte Kristallisation aus der Schmelze deutlich niedrigere Restgasmengen aufbereitet werden müssen, als beispielsweise unter Wirbelschicht-Verfestigungsverfahren mit NH₃-Gas oder festen Melamin und Inertstoffen als Kristallisationshilfsmittel.

Unabhängig von der Restgasaufbereitung ermöglicht das vorliegende Verfahren bereits im Vorfeld die Rückführung aller HD-Ammoniakgase wie, NH₃-Reaktionsgas aus dem Sekundärreaktor, NH₃-Aiginggas (falls die Aigingstufe angewandt wird) wieder in den Snytheseteil zu verwenden. Je nach gewähltem Systemdruck können die letzten zwei Gase innerhalb bevorzugterweise im Syntheseteil zur CO₂-Stripping verwertet werden (Fig. Nr. 2).

Falls das gewählte Systemdruck erlaubt, können hier auch die NH₃-Schutzgase aus dem Fallfilm-Vorkristallisator und Schmelzeraffination (in der Größenordnung von 200 - 300 kg/h) gemeinsam mit dem Luttengas aus der Kristallisation in die Offgaskolonne umgeleitet werden.

Zu der erfindungsgemäßen Restgasaufbereitung nach Fig. 3 können lediglich das Restgas aus der Verfestigungskammer (sog. Luttengas inkl. Perlgas des Schmelzekühlers, ca.350 kg/h) und NH₃/N₂ Desorptionsgase aus dem gewählten Deodorationsapparat (HD-Dreikammer-, -Fließbettdesorber, oder Wirbelschichtdeodorationsapparat). Die mit Melamin beladene Restgasleitungen werden zur Verhinderung der Belagbildung mantelseitig bis zum Fallfilm-Dephlegmator 70 beheizt. Das Kühlwasser zum Fallfilm-Dephlegmator 70 wird über eine kontinuirliche Qualitätsmessung (z. B. Trübungsmessung) in Abstimmung mit inertgashaltigem Restgas so geregelt, daß eine festsoffreie Melminlösung in flüssigem Ammoniak in dem erzeugten einphasigen Kondensat bis ca. 10 - 80% des Kristallisationspunktes der Lösung erreicht wird.

Durch Zumischung des reinen flüssigen NH₃ aus dem Kopfkondensator 72 in den Verteiler des Dephlegmators 70 unter zusätzlicher Kaskadenregelung wird das Unterschreiten der Löslichkeitsgrenze überwacht und verhindert. Die an Inertgasen angereicherte und weitgehend melaminfreie Gasphase des Dephlegmators 70 wird hier dem Verstärkungsteil der Restgas-Rektifizierkolonne 71 zugeführt, während die Melaminlösung in einphasigem Aggregatzustand über den Abtriebsteil zum Kolonnensumpf abfließt. Bis zum Sumpfteil erreicht das NH_{3,fl} weitgehend die Zusammensetzung einer Melamin gesättigten NH_{3,fl} Lösung. Der Sumpfteil der Kolonne wird durch Reboiler 73 unter kontinuierlicher Qualitätsmessung so gehalten, daß der Sättigungsgrad der Melaminlösung bei einem maximalen Kristallisationspunkt von 95 - 100% gehalten wird. Dadurch ist gewährleistet, daß in der gesamten Kolonne keine Melaminsuspension entsteht. Somit wird die Rektifizierkolonne 71 im Zweiphasengebiet mit Melaminlösung und NH₃-Gas feststoffrei betrieben.

Die aufkonzentrierte Melaminlösung ohne ausgefallenen Feststoff im Sumpfteil der Rektifizierkolonne wird kontinuierlich einem Dünnschichtkristallisator 74 zugeleitet. In Dünnschichtkristallisator 74 erfolgt nun die Fraktionierung der aufkonzentrierten Melaminlösung zur festen Melaminfraktion und NH₃-Brüden, die wieder zwecks Wärmeverwertung in die Rektifizierkolonne 71 zurückgeführt werden. Aus dem Dünschichtkristalllisator 74 gewinnt man das feste geruchlose Melamin zurück.

Das zurückgewonnene und geruchlose Melamin aus dem Dünnschichtkristallisator 74 kann wahlweise im Zwischenbunker (nach der Melamindeodoration 43) verschnitten und nach der Entspannung über die Mahlwerke homogen mit Hauptprodukt vermischt werden.

**Tab. 1 Figuren des Verfahrens**

| | |
|---|---|
| Figuren-Nr. | Bezeichnung |
| Figur Nr. 1 | Verfahrensübersicht mit Darstellung der flexiblen Produktionslinie |
| Figur Nr. 2 | Syntheseteil mit Rohstoffvorbehandlung |
| Figur Nr. 3 | Verfestigungseinheit mit Produktfinalisierung und Restgasaufbereitung |
| Figur Nr. 4 | Alternative Aigingstufe mit Reinigung der Melaminschmelze und zwei Raffinationsstufen |
| Figur Nr. 5 | Offgasincineration in Überdruckvariante |

**Tab. 2 Legende zu den Bezugsnummern in den Figuren 1 bis 5**

| Nr. | Bezeichnung |
|---|---|
| 1 | Melamin-Primärreaktor (Beheizung elektrisch oder mit Salzschmelze) |
| 2 | Abscheider |
| 3 | 4-Zonen-Offgaskolonne |
| 4 | Polytropischer CO2-Stripper mit indirekter Beheizung |
| 5 | Heizrohre mit makropöroser Packungsmatrix des Sekundärreaktors |
| 6 | Melamin-Sekundärreaktor |
| 7 | Böden der Offgaskolonne mit Kühlrohren |
| 8 | Tropfenabscheider der Offgaskolonne |
| 9 | Böden der Offgaskolonne ohne Kühlrohre |
| 10 | Polytropische Preabsorptionszone der Offgaskolonne |
| 11 | Adiabatische Aerosolabscheidezone |
| 12 | Autotherme Offgaszone für Offgaswäsche und Offgaskühlung |
| 13 | Polytropische Sedimentationszone mit indirekter Beheizung zur thermischen Einstellung des Syntheseharnstoffes |
| 14 | Syntheseharnstoffpumpe |
| 15 | Druckerhöhungspumpe der Melaminschmelze für Aigingstufe |
| 16 | Reinigungsstufe |
| 17 | Aigingreaktor (eventuell für polytripische Betriebsbedingung) |
| 18 | Fallfilm-Vorkristallisation unter NH3-Atmosphäre |
| 19 | Vorlagebehälter der Melaminschmelze für diskontinuierliche Schmelzeraffination |
| 20 | Fallfilm-Produktkristallisator unter NH3-Atmosphäre |
| 21 | Heiz- und Kühlwärmeaustauscher für diskontinuierliche Schmelzeraffination |
| 22 | Vorlage für verunreinigte Melaminschmelze (Aigingbehälter bei Regenerationsschritt) |
| 23 | Melminschmelze-Kreislaufpumpe |
| 24 | Wärmeträger-Kreistaufpumpe |
| 25 | Verteiler der Melaminschmelze |
| 26 | Verteiler und Sammler des Wärmeträgers |
| 27 | Melaminschmelzekühler und NH3-Sättiger |
| 28 | Kühlrohre mit Header |
| 29 | Statische Mischungselemente in der Kühl- und Relaxationsstrecke |
| 30 | Düse für NH3-Perlgas |
| 31 | Erweiterte Mantelkühlung entlang der Beharrungsstrecke |
| 32 | Entspannungsgefäß |
| 33 | Zwillingswalzenkristallisator |
| 34 | Kristallisierwalze (alternativ Kühlband des Bandkristallisators) |
| 35 | Schabmesser oder Schabwalze |
| 36 | Nibbler |
| 37 | Lutte |
| 38 | Dom zur Gasableitung aus den Zonen des HD-Deodorationsapparat |
| 39 | Beheizte Taumel mit Beschaufelung zur Grobgutförderung |
| 40 | Statische Heizringplatte |
| 41 | Labyrintdichtung oder Gleitringdichtung mit N₂-Sperrgas bzw. N₂- oder NH₃-Schmiergas |
| 42 | Getriebesatz und Antrieb |
| 43 | HD-Dreikammer-Deodorationsapparat |
| 44 | Buffer zur Zonentrennung mit Produktfördertaumel |
| 45 | Halbmond-Filterkerzen zur Gasverteilung |
| 46 | Statikmischer für Gemischgas Verdrängungsdesorption |
| 47 | Primärluftfilter |
| 48 | Maschinensatz mit Verdichter, Expanderturbine, Dampfturbine oder elektrischem Antrieb |
| 49 | Luftstripper für Schwachlaugen |
| 50 | Statikmischer für Verbrennungsluft/Offgas mit Mantelheizung |
| 51 | Entspannungsventil |
| 52 | Erdgas-Anfahrverdichter für Überdruckincineration |
| 53 | Incinerationreaktor |
| 54 | Erdgaslanzen |
| 55 | Verteiler und Gleichrichter für Verbrennungsmedium |
| 56 | Katalysatorbett |
| 57 | Wärmeaustauscher zur Wärmeverwertung (Salzschmelze-Erhitzer, Wärrneöl-Erhitzer, Wärmeöl-Verdampfer, Dampferzeuger) |
| 58 | Statikmischer für NH3-Injektion |
| 59 | SKR |
| 60 | Economiser |
| 61 | LUVO |
| 62 | Kamin |
| 63 | Adsorberkolonne für NH3,fl. Entölung |
| 64 | Dehydratationsadsorber für NH3,fl. |
| 65 | Boosterpumpe |
| 66 | NH3,fl HD-Pumpe |
| 67 | Adsorberkolonne für Entölung der Harnstoffschmelze |
| 68 | Statikmischer für NH3-Stabilisierungsgas (evntuell mit Kühlrohren) |
| 69 | Harnstoffschmelze HD-Pumpe |
| 70 | Fallfilm-Dephlegmator für Restgase |
| 71 | NH3-Restgasrektifizierkolonne |
| 72 | Kopfkondensator mit Rückfluß in den Verstärkungsteil und Dephlegmator |
| 73 | Reboiler zum Lastausgleich |
| 74 | Melamin-Donnschichtkristallisator mit Brüdenrückführung |
| 75 | Kühl- und Sättigungsstrecke des Melaminschmelzekühlers |
| 76 | Relaxationsstrecke zur Schmelzestabilisation |
| 77 | Kühl- und Sättigungsstrecke entlang der Beharrungsstrecke bis zur Produktaufgabe auf die Kristallisatorkühlfläche |
| 78 | NH₃ stabilisierte Aufheizzone zur thermischen Desorption des Melamingrobgutes (1. Zone des HD-Deodorationsapparates) |
| 79 | Thermische Desorptionszone unter simultanem Gemischgasstripping des Grobgutes (2. Zone des HD-Deodorationsapparates) |
| 80 | Kühlzone des Grobgutes unter N₂-Stripping (3. Zone des HD-Deodorationsapparates) |

**Tab. 3 Mediennummer in Ausführungsbeispiel**

| Nr. | Bezeichnung |
|---|---|
| M1 | Frische Hamstoffschmelze |
| M2 | Stabilisierte Harnstoffschmelze |
| M3 | Entölte Harnstoffschmelze nach HD-Pumpe zur Offgaskolonne |
| M4 | Thermisch eingestellter Syntheseharnstoff mit suspendiertem Melamin zum Primärreaktor |
| M5 | Inerten freies NH_{3,fl.} (evtl. über Boosterpumpe) zur Vorbehandlung |
| M6 | Entöltes NH_{3,fl.} |
| M7 | Dehydratisiertes NH_{3,fl.} nach der HD-Pumpe zum Verdampfer und Überhitzer |
| M8 | Frisches NH₃ Gas für diverse Einsätze |
| M9 | NH₃-Zusatzgas zum Primärreaktor |
| M10 | NH₃-Reaktionsgas zum Sekundärreaktor |
| M11 | Frisches NH₃-Strippgas zum CO₂-Stripper |
| M12 | Melaminschmelze aus dem Primärreaktor und Abscheider zum CO₂-Stripper |
| M13 | CO₂-gestrippte Melaminschmelze zum Sekundärreaktor |
| M14 | Melaminschmelze aus dem Sekundärreaktor, Melaminschmelze zur Verfestigungseinheit für Produkt der Güteklasse III |
| M15 | Offgas des Primärreaktors |
| M16 | Offgas des Sekundärreaktors zum CO₂-Stripper |
| M17 | Abgas des CO₂-Strippers |
| M18 | Syntheseoffgase zur 4-Zonen-Offgaskolonne |
| M19 | NH₃-Recyclgas zur Verwertung in CO₂-Stripper |
| M20 | Gereinigtes Offgas zur Kondensation oder Incineration |
| M21 | NH₃-Stabilisiergas zur Harnstoffschmelze |
| M22 | Vorgereinigte Melaminschmelze nach der Fallfilm-Vorkristallisation, Melaminschmelze zur Verfestigungseinheit für Produkt der Güteklasse II |
| M23 | Gereinigte Melaminschmelze nach der Raffination, Melaminschmelze zur Verfestigungseinheit für Produkt der Güteklasse I |
| M24 | NH₃ partiell gesättigte vorgekühlte Melaminschmelze über Entgasergefäß zum Produktkristallisator |
| M25 | Poröses Grobgutmelamin zur HD-Deodoration |
| M26 | Geruchfreies, pH-neutrales Grobgut zu Zwischenentspannungsbehältem |
| M27 | NH₃-Perlgas zur partiellen Sättigung der Melaminschmelze im Schmelzekühler |
| M28 | NH₃-Schutzgas für die Aufheizzone zur thermischen Desorption des HD-Deodorationsapparates |
| M29 | N₂/NH₃-Strippgasgemisch zur thermischen Verdrängungsdesorption des HD-Deodorationsapparates |
| M30 | N₂-Gas zur Verdrängungsdesorptionzone und Kühlzone des HD-Deodorationsapparates |
| M31 | Melaminhältiges NH₃-Restgas von der Lutte zur Aufbereitung |
| M32 | Desorptionsgase der Deodorationsstufe zur Restgasaufbereitung |
| M33 | NH_{3,fl} Melaminlösungskondensat des Dephlegmators |
| M34 | NH₃/Inertengase nach dem Dephlegmator zum Verstärkungsteil der Rektifizierkolonne |
| M35 | Aufkonzentrierte Melaminlösung zum Dünnschichtkristallisator |
| M36 | Melaminpulver nach Dünnschichtkristallisator |
| M37 | NH₃-Brüdenrückführung zur Verwertung |
| M38 | NH₃-Dampf zum Kopfkondensator |
| M39 | Reines NH_{3,fl} Prozeßkondensat zum Vorlagetank |
| M40 | NH_{3,fl}-Verdünnungsmittel für Dephlegmatorkondensat |
| M41 | Inertenableitung des Kopfkondensators |
| M42 | Gereinigte Melaminschmelze |
| M43 | Melaminschmelze nach der Aigingstufe |
| M44 | Vorgereinigte Melaminschmelze zur 2. Raffinationsstufe |
| M45 | Rücklauf der Melaminschmelze mit angereicherten Nebenverbindungen zum |
| M46 | NH₃-Schützgas zu den Vorlagebehältern und Kristallisator der Raffinationsstufen, Aiginggas während der intermittierenden Schmelzebehandlung |
| M47 | NH₃ Recyclgas aus der Aigingstufe zum CO₂-Stripper |
| M48 | NH₃-Atmosphärengas zur Offgaskolonne oder Restgasaufbereitung |
| M49 | HD-NH₃-Gas zur Aigingstufe |
| M50 | Primärluft zur Incineration |
| M51 | Sekundärluft zur Stripping und Incineration |
| M52 | Abluft aus div. Apparaten, Behältern und Inertenableitungen |
| M53 | Verbrennungsluft nach Gebläse oder Axialverdichter |
| M54 | Offgas-Verbrennungsluft-Gemisch |
| M55 | NOₓ-reiches Abgas nach Incineration |
| M56 | NH₃-hältiges Offgas als Injektionsgas für SKR |
| M57 | Gereinigtes Verbrennungsabgas zum Expander |
| M58 | Entspanntes gereinigtes Abgas zum LUVO |
| M59 | Gereinigtes Abgas zum Kamin |
| M60 | NH₃/CO₂-hältige Schwachlaugen der Melamin- und Harnstoffanlage zum Luftstripper |
| M61 | Gestripptes NH₃-freies Abwasser zum Kanal |
| M62 | Sauerkondensat zur Neutralisation |
| M63 | Erdgas für Anfahrphase und Lastausgleich |

**Tab. 4 Betriebsmittelnummer im Ausführungsbeispiel**

| Nr. | Bezeichnung |
|---|---|
| AM1 | Salzschmelze Vorlauf zu Primär- und Sekundärreaktor |
| AM2 | Salzschmelze Auslauf von Primär- und Sekundärreaktor |
| AM3 | Wärmeträgereintritt in den polytropischen CO₂-Stripper |
| AM4 | Wärmeträgeraustritt vom polytropischen CO₂-Stripper |
| AM5 | Wärmeaufnehmendes Medium zur Preabsorptionszone der Offgaskolonne (z. B. Wärmeölträger, Kesselwasser vom Dampfkessel) |
| AM6 | Erhitztes Medium von der Preabsorptionszone der Offgaskolonne (z. B. Wärmeölträger, Kesselwasser zur Dampftrommel) |
| AM7 | Zulauf des Wärmeträgers zur thermischen Einstellung des Syntheseharnstoffes in der Sedimentationszone der Offgaskolonne |
| AM8 | Ablauf des Wärmeträgers aus der Sedimentationszone der Offgaskolonne |
| AM9 | Zulauf des Heizmittels zur intermittierenden Regeneration der Dehydratationsadsorberkolonne |
| AM10 | Ablauf des Heizmittels von der intermittierenden Regeneration der Dehydratationsadsorberkolonne |
| AM11 | Eintritt des Wärmeträgers in die Kühlsektion des Melaminschmeizekühlers und NH₃-Sättiger |
| AM12 | Austritt des Wärmeträgers aus der Kühlsektion des Melaminschmelzekühlers und NH₃-Sättiger |
| AM13 | Heizmediumeintritt in die Aufheizzone und thermische Desorptionzone des HD-Deodorationsapparates |
| AM14 | Heizmediumaustritt von der Aufheizzone und thermischer Desorptionzone des HD-Deodorationsapparates |
| AM15 | Kühlmitteleintritt in die N₂-Verdrängungsdesorptionszone und Kühlzone des HD-Deodorationsapparates |
| AM16 | Kühlmittelaustritt von der N₂-Verdrängungsdesorptionszone und Kühlzone des HD-Deodorationsapparates |
| AM17 | Kühlwassereintritt in den Dephlegmator |
| AM18 | Kühlwasseraustritt vom Dephlegmator |
| AM19 | Heizmittel zum Reboiler der NH3-Restgasrektifizierkolonne |
| AM20 | Heizmittel zum Dünnschichtkristallisator |
| AM21 | Kühlwasser (Sekundärkühlkreislauf) des Kopfkondensators |
| AM22 | Wärmeträgervorlauf zur partiellen Kristallisation in Fallfilm-Vorkristallisator |
| AM23 | Wärmeträgerauslauf von der partiellen Kristallisation in Fallfilm-Vorkristallisator |
| AM24 | Kühl- und Heizmedium der 2. Raffinationsstufe |

## Patentansprüche

1. Verfahren zur kontinuierlichen Herstellung von Melamin, bei dem durch nichtkatalytische Hochdruckpyrolyse (HD-Pyrolyse) von Harnstoff eine Melaminschmelze erhalten wird, die nach einer oder mehreren Raffinationsstufen (18, 20) und indirekter Melaminschmelzekühlung unter simultaner NH₃-Partialsättigung durch trockene Aufbereitung mittels direkter Hochdruck-Schmelzekristallisation (HD-Schmelzekristallisation) (33) ohne Kristallisationshilfsmittel unter NH₃-Atmospäre bei indirekter Kühlung (34) der Schmelze mit einem Kühlmittel oder einem Kältemittel verfestigt wird, aus dem erhaltenen Produkt unter Hochdruckbedingungen NH₃ entfernt wird und das Produkt zur beliebigen Korngrößenverteilung in eine oder mehrere, vorzugsweise bis in vier Güteklassen finalisiert wird, wobei die eine oder mehreren Güteklassen aus einer bei der nichtkatalytischen HD-Pyrolyse erhaltenen Melaminschmelze und/oder aus einer oder mehreren ein- oder mehrfach raffinierten Melaminschmelzen und/oder durch wäßrige Umkristallisation von unter Überdruck verfestigtem Melamin erhalten werden.

2. Verfahren nach Anspruch 1, **dadurch gekennzeichnet, daß** die Melaminschmelze nach einem Primärreaktor (1) und einem Abscheider (2) in einer polytropischen CO₂-Stripperkolonne (4) gestrippt und einem Sekundärreaktor (6) bis zum vollständigen Umsatz von Harnstoff und Zwischenprodukten zugeführt wird.

3. Verfahren nach Anspruch 1 oder 2, **dadurch gekennzeichnet, daß** die heißen melaminhaltigen Reaktionsgase aus dem Primärreaktor (1) und Strippergas aus der CO₂-Stripperkolonne (4) in eine 4-Zonen-Offgaskolonne (3) mit polytropischer Preabsorptionszone (10), adiabatischer Aerosolabscheidezone (11), autothermer Waschzone (12) und polytropischer Eindickerzone (13) mit frischer Harnstoffschmelze abgekühlt und von Melamin gereinigt werden.

4. Verfahren nach einem oder mehreren der Ansprüche 1 bis 3, **dadurch gekennzeichnet, daß** nach der HD-Pyrolyse die Melaminschmelze in einer Reinigungsstufe (16) gereinigt und durch eine Aigingstufe (15, 17) weiterbehandelt wird.

5. Verfahren nach einem oder mehreren der Ansprüche 1 bis 4, **dadurch gekennzeichnet, daß** die Melaminschmelze aus dem Sekundärreaktor (6) durch eine zweistufige Raffination (18, 20) der Schmelze von den höher und niedriger als Melamin schmelzenden Verunreinigungen gereinigt wird.

6. Verfahren nach einem oder mehreren der Ansprüche 1 bis 5, **dadurch gekennzeichnet, daß** die Melaminschmelze vor der HD-Schmelzekristallisation (33) durch kontrollierte indirekte Kühlung (28) unter kontrollierter simultaner NH₃-Partialsättigung (30, 75) bis zum NH₃-Partialdruck abhängigen Kristallisationspunkt (Stockungspunkt) der Schmelze vorgekühlt wird.

7. Verfahren nach einem oder mehreren der Ansprüche 1 bis 6, **dadurch gekennzeichnet, daß** die Melaminschmelze durch indirekte Kühlung (34) mit einem Kühlmittel oder einem Kältemittel unter HD-Bedingungen und NH₃-Atmosphäre ohne Kristallisationshilfsmittel mittels eines Bandkristallisators, eines Einwalzenkristallisators oder besonders vorteilhaft mit einem Zwillingswalzenkristallisator zu einem porösen Melamingefüge verfestigt wird.

8. Verfahren nach einem oder mehreren der Ansprüche 1 bis 7, **dadurch gekennzeichnet, daß** das verfestigte poröse Melamin zur Bereitstellung eines pH-neutralen und NH₃ geruchfreien Produktes durch Deodoration unter HD-Bedingungen und NH₃/N₂-Gemischgas in einem Dreikammer-Deodorationsapparat (43) oder dreizonigen Fließbettdeodorationsapparat oder zweizonigen Wirbelschichtapparat behandelt wird, wobei die Deodoration bei einer höheren Temperatur als die Verfestigung des Melamins durchgeführt wird.

9. Verfahren nach einem oder mehreren der Ansprüche 1 bis 8, **dadurch gekennzeichnet, daß** die Herstellung von Melamin derart flexibel ausgestaltet werden kann, daß vier Produktqualitäten nach Spezifikation derart bereitgestellt werden können, daß das Produkt in der Güteklasse III durch die Verfestigung der Melaminschmelze nach dem Sekundärreaktor (6), Produktklasse II nach der ersten Raffinationsstufe (18), Produktklasse I durch die Verfestigung der Melaminschmelze nach der zweiten Raffinationsstufe (20) oder eine Sonderqualität durch wäßrige Umkristallisation des unter Überdruck verfestigten Melamins her gestellt wird.

10. Verfahren nach einem oder mehreren der Ansprüche 1 bis 9, **dadurch gekennzeichnet, daß** die Offgase nach der Offaskolonne (3) einer Incinerationstufe unter Normaldruck oder Überdruck zugeführt werden, wobei die gewonnene Wärme für interne Zwecke in der Anlage oder für externe Einsätze, vorzugsweise für die Dampferzeugung, den Antrieb der Arbeitsmaschinen oder-für die Erzeugung elektrischer Energie, verwertet wird.

11. Verfahren nach einem oder mehreren der Ansprüche 1 bis 10, **dadurch gekennzeichnet, daß** die Einsatzstoffe für die Melaminherstellung, Harnstoff und Ammoniak, vor der Melaminsynthese entölt (67) werden, wobei der Ammoniak zusätzlich dehydratisiert wird.

12. Verfahren nach einem oder mehreren der Ansprüche 1 bis 11, **dadurch gekennzeichnet, daß** die melaminhaltigen HD-Restgase aus der Anlage entweder der Incinerationstufe zugeführt werden oder durch Fraktionierung in eine reine NH₃-Fraktion und Pulvermelamin zerlegt werden.

## Claims

1. A method for the continuous manufacture of melamine, in which a melamine melt is obtained by non-catalytic high pressure pyrolysis (HD pyrolysis) of urea, said melamine melt being solidified after one or more refinement steps (18, 20) and after indirect melamine melt cooling with a simultaneous partial NH₃ saturation by dry conditioning by means of direct high pressure melt crystallisation (HD melt crystallisation) (33) without any crystallisation aids in an NH₃ atmosphere with indirect cooling (34) of the melt by a coolant or refrigerant, in which NH₃ is removed from the product obtained under high pressure conditions and in which the product is finalised into one or more quality classes, preferably into up to four quality classes, for the desired grain size distribution, wherein the one or more quality classes are obtained from a melamine melt obtained in the non-catalytic HD pyrolysis and/or from one or more simply or multiply refined melamine melts and/or by aqueous recrystallisation of melamine solidified under excess pressure.

2. A method in accordance with claim 1, **characterised in that** the melamine melt is stripped in a polytropic CO₂ stripper column (4) subsequent to a primary reactor and a separator (2) and is supplied to a secondary reactor (6) up to the complete conversion of urea and intermediate products.

3. A method in accordance with either of claims 1 or 2, **characterised in that** the hot reaction gases containing melamine from the primary reactor (1) and stripper gas from the CO₂ stripper column (4) are cooled and cleaned of melamine in a 4-zone off-gas column (3) with a polytropic pre-absorption zone (10), an adiabatic aerosol separation zone (11), an autothermal washing zone (12) and a polytropic thickener zone (13) with a fresh urea melt.

4. A method in accordance with one or more of the claims 1 to 3, **characterised in that** the melamine melt is cleaned in a cleaning stage (16) and further treated in an aging stage (15, 17) after the HD pyrolysis.

5. A method in accordance with one or more of the claims 1 to 4, **characterised in that** the melamine melt from the secondary reactor (6) is cleaned from pollutants melting higher and lower than melamine by a two-stage refinement (18, 20) of the melt.

6. A method in accordance with one or more of the claims 1 to 5, **characterised in that** the melamine melt is pre-cooled before the HD melt crystallisation (33) by a controlled indirect cooling (28) in controlled simultaneous partial NH₃ saturation (30, 75) up to the crystallisation point (stagnation point) of the melt dependent on the partial NH₃ pressure.

7. A method in accordance with one or more of the claims 1 to 6, **characterised in that** the melamine melt is solidified to form a porous melamine structure by indirect cooling (34) using a coolant or a refrigerant under HD conditions and in an NH₃ atmosphere without crystallisation aids by means of a band crystalliser, a single roll crystalliser or, particularly advantageously, using a twin roll crystalliser.

8. A method in accordance with one or more of the claims 1 to 7, **characterised in that** the solidified porous melamine is treated for the provision of a pH neutral and NH₃ odour free product by deodorisation under HD conditions and in an NH₃/NH₂ mixed gas in a three-chamber deodorisation apparatus (43) or in a three-zone fluidised bed deodorisation apparatus or in a two-zone fluidised bed apparatus, with the deodorisation being carried out at a higher temperature than the solidifying of the melamine.

9. A method in accordance with one or more of the claims 1 to 8, **characterised in that** the manufacture of melamine can be configured so flexibly that four product qualities can be provided by specification such that the product in quality class III is manufactured by the solidification of the melamine melt after the secondary reactor (6); product class II is manufactured after the first refining stage (18); product class I by the solidification of the melamine melt after the second refining stage (20) or a special quality is manufactured by aqueous recrystallisation of the melamine solidified under excess pressure.

10. A method in accordance with one or more of the claims 1 to 9, **characterised in that** the off-gases are supplied to an incineration stage under normal pressure or excess pressure after the off-gas column (3), wherein the heat gained is utilised for internal purposes in the plant or for external uses, preferably for steam generation, for the drive of the working machines or for the generation of electrical energy.

11. A method in accordance with one or more of the claims 1 to 10, **characterised in that** the feed materials for the melamine manufacture, urea and ammonia, are deoiled (67) before the melamine synthesis, with the ammonia additionally being dehydrated.

12. A method in accordance with one or more of the claims 1 to 11, **characterised in that** the HD residual gases containing melamine from the plant are either supplied to the incineration stage or are broken down by fractionating into a pure NH₃ fraction and powder melamine.

## Revendications

1. Procédé pour la fabrication continue de mélamine permettant d'obtenir de la mélamine en fusion au moyen d'une pyrolyse haute pression (pyrolyse HP) non catalytique d'urée, laquelle mélamine, après une ou plusieurs étapes de raffinage (18, 20) et refroidissement indirect de la mélamine en fusion, est solidifiée sous saturation simultanée partielle de NH₃ à l'aide d'un traitement à sec par cristallisation de la fusion à haute pression (cristallisation de la fusion HP) (33) sans adjuvant de cristallisation sous une atmosphère NH₃ par refroidissement indirect (34) de la fusion au moyen d'un agent de refroidissement ou d'un agent réfrigérant ; de supprimer le NH₃ du produit obtenu dans des conditions sous haute pression ; et de finaliser le produit en vue d'obtenir la répartition granulométrique au choix dans une ou plusieurs classes de produits, le nombre de classes s'élevant de préférence à quatre au maximum, la ou les classes de produits étant obtenues à partir de la mélamine en fusion produite lors de la pyrolyse HP non catalytique et/ou à partir de la mélamine en fusion raffinée une ou plusieurs fois et/ou de la mélamine solidifiée par la recristallisation aqueuse dans des condition de surpression.

2. Procédé selon la revendication 1, **caractérisé en ce que** la mélamine en fusion est extraite par vapeur après un premier réacteur primaire (1) et un séparateur (2) dans une colonne de stripping au CO₂ polytropique (4) et est ajoutée à un réacteur secondaire (6) jusqu'à obtenir un rendement complet de l'urée et des produits intermédiaires.

3. Procédé selon la revendication 1 ou 2, **caractérisé en ce que** les gaz de réaction chauds présentant une teneur en mélamine sortant du réacteur primaire (1) et le gaz de stripping sortant de la colonne de stripping au CO₂ (4) sont refroidis dans une colonne de gaz de dégagement à 4 zones (3) présentant une zone de préabsorption polytropique (10), une zone de séparation aérosol adiabatique (11), une zone de lavage autothermique (12) et une zone de condensation polytropique (13) avec une fusion récente de l'urée et sont purifiés par la mélamine.

4. Procédé selon une ou plusieurs des revendications 1 à 3, **caractérisé en ce qu'**après la pyrolyse HP, la mélamine en fusion est purifiée lors d'une étape de purification (16) et son traitement se poursuit par une étape de vieillissement (15, 17).

5. Procédé selon une ou plusieurs des revendications 1 à 4, **caractérisé en ce que** la fusion de mélamine sortant du réacteur secondaire (6) est purifiée des impuretés fusionnant à une température inférieure et supérieure à celle de la mélamine au moyen d'un raffinage en deux temps (18, 20) de la fusion.

6. Procédé selon une ou plusieurs des revendications 1 à 5, **caractérisé en ce que** la mélamine en fusion est préréfroidie avant la cristallisation de la fusion HP (33) au moyen d'un refroidissement indirect contrôlé (28) sous saturation partielle et simultanée de NH₃ contrôlée (30, 75) jusqu'au point de cristallisation de la fusion dépendant de la pression partielle du NH₃.

7. Procédé selon une ou plusieurs des revendications 1 à 6, **caractérisé en ce que** la mélamine en fusion est solidifiée en une structure de mélamine poreuse par refroidissement indirect (34) à l'aide d'un agent de refroidissement ou d'un agent réfrigérant dans des conditions HP et sous une atmosphère de NH₃ sans adjuvant de cristallisation au moyen d'un cristallisateur à bande, d'un cristallisateur à cylindre unique et de manière particulièrement avantageuse, d'un cristallisateur à double cylindre.

8. Procédé selon une ou plusieurs des revendications 1 à 7, **caractérisé en ce que** la mélamine poreuse solidifiée est manipulée pour la préparation d'un produit à pH neutre exempt d'odeur de NH₃ par désodorisation dans des conditions HP et par un gaz de mélange NH₃/N₂ dans un appareil de désodorisation (43) à trois chambres ou un appareil de désodorisation sur lit fluidisé à trois zones ou un appareil sur lit fluidisé à deux zones, la désodorisation étant réalisée à une température supérieure à celle de la solidification de la mélamine.

9. Procédé selon une ou plusieurs des revendications 1 à 8, **caractérisé en ce que** la production de mélamine peut être aménagée de façon flexible, **en ce que** quatre qualités de produits peuvent être préparées conformément à la description de sorte à fabriquer le produit de la classe de produit III par la solidification de la mélamine en fusion après le réacteur secondaire (6), le produit de classe II après la première étape de raffinage (18), le produit de classe I par la solidification de la mélamine en fusion après la deuxième étape de raffinage (20) et une qualité spéciale obtenue par la recristallisation aqueuse de la mélamine solidifiée dans des condition de surpression.

10. Procédé selon une ou plusieurs des revendications 1 à 9, **caractérisé en ce que** les gaz de dégagement sont acheminés après la colonne gaz de dégagement (3) vers une étape d'incinération sous pression normale ou surpression, la chaleur obtenue étant recyclée à des fins internes dans l'installation ou pour des utilisations externes, de préférence pour la production de vapeur, l'entraînement de machines de travail ou pour la production d'énergie électrique.

11. Procédé selon une ou plusieurs des revendications 1 à 10, **caractérisé en ce que** les matières chargées pour la production de mélamine, l'urée et l'ammoniaque, sont déshuilées (67) avant la synthèse de la mélamine, l'ammoniaque étant en plus déshydratée.

12. Procédé selon une ou plusieurs des revendications 1 à 11 **caractérisé en ce que** les gaz résiduels HP contenant de la mélamine sortant de l'installation sont soit acheminés vers l'étape d'incinération, soit décomposés par fractionnement en une fraction NH₃ pure et en poudre de mélamine.
